# EUROPEAN PATENT SPECIFICATION

(11) **EP 1 998 775 B1**
(45) Date of publication and mention of the grant of the patent: **19.05.2010**
(21) Application number: 07726623.7
(22) Date of filing: 03.03.2007
(51) Int. Cl.: A61K 31/4725, A61P 25/28, A61P 25/24, A61P 25/18, A61P 25/16, C07D 217/04

(54) **BIS1,2,3,4-TETRAHYDROISOQUINOLINE DERIVATIVES AND THEIR USES AS PHARMACEUTICALS**
BIS-1,2,3,4-TETRAHYDROISOCHINOLINDERIVATE UND IHRE VERWENDUNG ALS PHARMAZEUTIKA
UTILISATION DE DERIVÉS DE 1,2,3,4 TETRAHYDROISOQUINOLINE COMME BLOCKEURS DU CANAL SK

(30) Priority: 17.03.2006 EP 06111355
(43) Date of publication of application: 10.12.2008
(73) Proprietor: Université de Liège, 4031 Angleur (BE)
(72) Inventor: GRAULICH, Amaury, B-4357 Donceel (BE); LIEGEOIS, Jean-Francois, B-4000 Liege (BE); MOREAU, Jacqueline, B-4000 Liege (BE); SEUTIN, Vincent, B-4020 Liege (BE)
(86) International application number: PCT/EP2007/052029
(87) International publication number: WO 2007/107442

(56) References cited:
- WO-A-2005/121126
- US-A- 5 760 230
- US-A- 5 874 438
- US-B1- 6 174 897
- GRAULICH A ET AL: "Synthesis and biological evaluation of N-methyl-laudanosine iodide analogues as potential SK channel blockers" BIOORGANIC & MEDICINAL CHEMISTRY, ELSEVIER SCIENCE LTD, GB, vol. 13, no. 4, 15 February 2005 (2005-02-15), pages 1201-1209, XP004724941 ISSN: 0968-0896
- GRAULICH AMAURY ET AL: "Synthesis and radioligand binding studies of bis-isoquinolinium derivatives as small conductance Ca(2+)-activated K(+) channel blockers." JOURNAL OF MEDICINAL CHEMISTRY 18 OCT 2007, vol. 50, no. 21, 18 October 2007 (2007-10-18), pages 5070-5075, XP002475420 ISSN: 0022-2623

## Description

The present invention relates to new bis 1,2,3,4-tetrahydroisoquinoline derivatives and their use as pharmaceuticals.
Particularly, the present invention relates to bis 1,2,3,4-tetrahydroisoquinoline derivatives and their use as SK channel blockers.
There are three families of calcium-activated potassium channels depending of their biophysical and pharmacological properties. They are called BK, IK and SK reflecting their large, intermediate and low conductance, respectively (Sah, Trends Neurosci. 1996, 19, 150-154 ; Vergara et al, Curr. Opin. Neurobiol. 1998, 8, 321-329).
Small conductance Ca²⁺-activated K⁺ (SK) channels underlie the prolonged postspike afterhyperpolarization (AHP) which plays an important role in modulating the firing rate and the firing pattern of neurones (Kolher et al, Science 1996, 273, 1709-1714 ; Stocker et al, Proc. Natl. Acad. Sci. USA 1999, 96, 4662-4667). Functional, pharmacological and structural data have suggested the existence of variants of the SK channel (Sah, Trends Neurosci. 1996, 19, 150-154). Indeed, three SK channel subtypes have been identified by DNA cloning, namely SK1, SK2 and SK3 (Kolher et al, Science 1996, 273, 1709-1714). In contrast to BK channels, SK channels are voltage-insensitive but are activated by an increase in the intracellular calcium concentration. The distribution of the SK channel subtypes was determined in the rat by using *in situ* hybridization and immunohistochemistry and it appeared that SK1 and SK2 subtypes are mostly expressed in the cortex and hippocampus (Stocker et al, Mol. Cell. Neurosci. 2000, 15, 476-493) while SK3 channels expression is higher in subcortical areas, especially in the monoamine cell regions e.g. substantia nigra, dorsal raphe and locus coeruleus.

Up to now, the said most potent SK channel blocker is Apamin. Apamin is extracted from the venom of the honey bee *Apis mellifera.* Apamin is a peptide constituted by 18 amino-acids: Cys-Asn-Cys-Lys-Ala-Pro-Glu-Thr-Ala-Leu-Cys-Ala-Arg-Arg-Cys-Gln-Gln-His-NH₂ and is a useful pharmacological tool.

However, its use is associated with many problems, arising from its toxicity, availability, cost, instability in biological media and poor bioavailability. Moreover, due to its toxicity, Apamin cannot be used for therapeutical treatment.

Dequalinium, a nonpeptidic ligand, has been described to presents some SK channel blocking properties and extensive structure-activity relationships studies have led to bis-quinolinium cyclophanes compounds (Campos Rosa et al, J. Med. Chem. 2000, 43, 420-431). Dequalinium and its derivatives are quaternary compounds.

Other quaternary compounds, such as, N-methyl-laudanosine (NML) and N-methyl-noscapine (NMN) have been reported to block SK channels (Scuvée-Moreau et al, J. Pharmacol. Exp. Ther. 2002, 302, 1176-1183)

Whilst Dequalinium, NML and NMN have medium potency blocking properties, their permanent ionization, due to the fact that they are quaternary compounds, is a serious drawback to cross the blood brain barrier. Therefore the possible therapeutic value of these compounds and their derivatives for the treatment of CNS diseases (i.e. diseases of the central nervous system) is therefore very poor.

We have now surprisingly found that new bis-1,2,3,4-tetrahydroisoquinoline of formula (I) exhibit significant affinity for SK channels, particularly at the Apamin-sensitive sites of SK channels.

The expression " SK channels" as used herein refers to SK₁, SK₂ and SK₃ channels. Therefore, it is understood that the present compounds of formula (I) may act as SK channels blockers or may interact selectively with one of the three subtypes (i.e. SK₁, SK₂ and SK₃).

US patent 2,744,901 describes bis- 1,2,3,4-tetrahydroisoquinoline derivatives which are useful as synthetic intermediates in the preparation of pharmaceutical compounds comprising bis-tétrahydro-1-isoquinolyl quaternary ammonium salts.

In a first aspect the present invention relates to bis-1,2,3,4-tetrahydroisoquinoline selected from the group consisting of
*1,3-Bis[1-(2-methyl-1,2,3,4-tetrahydrolisoquinolyl)]-propane hydrochloride*
*1,4-Bis[1-(2-methyl-1,2,3,4-tetrahydroisoquinolyl)]-butane hydrochloride*
*1,5-Bis[1-(2-methyl-1,2,3,4-tetrahydroisoquinolyl)]-pentane hydrochloride*
*1,3-Bis[1-(6,7-dimethoxy-2-methyl-1,2,3,4-tetrahydroisoquinolyl)]-propane hydrochloride*
*1,4-Bis[1-(6,7-dimethoxy-2-methyl-1,2,3,4-tetrahydroisoquinolyl)]-butane hydrochloride*
*1,5-Bis[1-(6,7-dimeihoxy-2-methyl-1,2,3,4-tetrahydroisoquinolyl)]-pentane hydrochloride*
*1,3-Bis[1-(6,7,8-trimethoxy-2-methyl-1,2,3,4-tetrahydroisoquinolyl)]-propane hydrochloride*
*1,4-Bis[1-(6,7,8-trimethoxy-2-methyl-1,2,3,4-tetrahydroisoquinolyl)]-butane hydrochloride*
*o-Bis[1-(2-methyl-1,2,3,4-tetrahydroisoquinolyl)]-xylene hydrochloride*
*m-Bis[1-(2-methyl-1,2,3,4-tetrahydroisoquinolyl)]-xylene hydrochloride*
*p-Bis[1-(2-methyl-1,2,3,4-tetrahydroisoquinolyl)]-xylene hydrochloride*
*o-Bis[1-(6,7-dimethoxy-2-methyl-1,2,3,4-tetrahydroisoquinoly*/*)]-xylene hydrochloride*
*m-Bis[1-(6,7-dimethoxy-2-methyl-1,2,3,4-tetrahydroisoquinolyl)]-xylene hydrochloride*
*p-Bis[1-(6,7-dimethoxy-2-methyl-1,2,3,4-tetrahydroisoquinolyl)]-xylene*
*o-Bis[1-(6,7,8-trimethoxy-2-methy*/*- 1,2,3,4-tetrahydroisoquinolyl)]-xylene hydrochloride*
*m-Bis(1-(6,7,8-trimethoxy-2-methyl-1,2,3,4-tetrahydroisoqurinolyl)]-xylene hydrochloride*
*p-Bis[1-(6,7,8-trimethoxy-2-methyl-1,2,3,4-tetrahydroisoquinolyl)]-xylene*
*1,3-bis[1-(2,8-dimethyl-1,2,3,4-tetrahydroisoquinolyl)]-propane hydrochloride*
*1,3-bis[1-(5-bromo-2-methyl-1,2,3,4-tetrahydroisoquinolyl)]-propane hydrochloride and*
*1,1'-bis[1-(2-methyl-1,2,3,4-tetrahydroisoquinolyl)]-dimethylelher*.

In a second aspect, the present invention relates to the use of bis-1,2,3,4-tetrahydroisoquinoline derivatives of formula (I), any stereoisomer thereof, including optical isomer or mixture of optical isomers thereof, including racemic mixture or any tautomeric or polymorphic form thereof and their pharmaceutically acceptable salt thereof 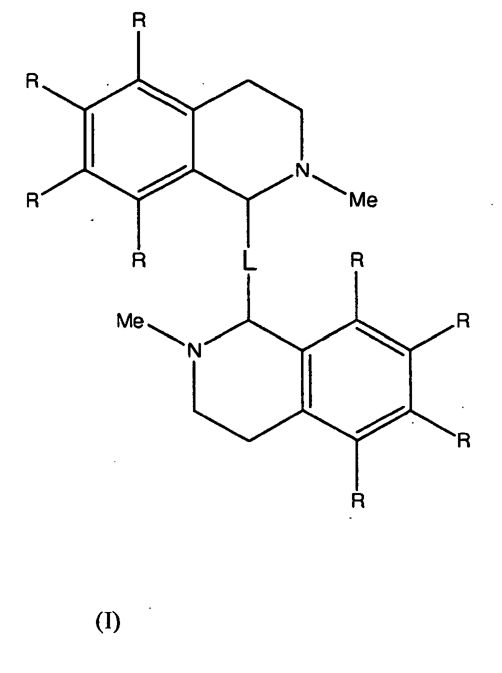 wherein
R which may be the same or different, represents hydrogen, hydroxy, C₁₋₁₂ alkyl, C₁₋₁₂ alkoxy, C₁₋₁₂ alkylthio, a C₁₋₁₂ alkylsulfoxide, a C₁₋₁₂ alkyl-sulfone, C₂₋₆ alkylenedioxy, an amino, an amido, an azido, nitro, C₁₋₁₂ alkylamino, C₁₋₁₂ alkylamido, C₁₋₁₂ alkylsulfonamido, a perhaloalkyl, a carboxyalkyl, a carboxy, a carbamide, dialkylamino, an aryl or halogen;
and L represents a G₁₋₁₂-alkyl, C₂₋₁₂alkenyl, a C₁₋₁₂alkynyl, an aryl, a diaryl, a cycloalkyl, an heterocycloalkyl, a cycloalkene, an heterocycloalkenyl, an heteroaryl, an ether, a thioether, a sulfoxide, a sulfone, a urea, a thiourea or a guanidine;
for the manufacture of a medicament for the treatment of diseases of the Central nervous system.

Bis 1,2,3,4-tetrahydroisoquinoline derivatives according to the present invention have generally two chiral centers and are therefore capable of occurring in optical isomeric forms. There are three distinct optical isomeric forms: the dextrorotatory, the levorotatory and the meso forms The present invention also refers to the use of all optical isomers of bis 12,3,4-tetrahydroisoquinoline derivatives, particularly to both enantiomers and meso analogue as well as their mixture, in particular their racemic mixtures.

The individual isomers can be prepared directly or by asymmetric or stereospecific synthesis or by conventional separation of optical isomers from the racemic mixtures such as fractional crystallization or chiral liquid chromatography.

The salts include pharmaceutically acceptable acid addition salts of the compounds of Formula I such as nitric, hydrochloric, hydrobromic, hydroiodic, phosphoric, phosphorous, sulfuric, and the like, as well as the salts derived from non-toxic organic acids, phenyl substituted alkanoic acids, hydroxy alkanoic acids, alkanedioic acids, aromatic acids, aliphatic and aromatic sulfonic acids, etc such salts thus include sulfate, pyrosulfate, bisulfate, sulfite, bisulfite, nitrate, phosphate, monohydrogenphosphate, dihydrogenphosphate, metaphosphate, pyrophosphate, chloride bromide, iodide, acetate, propionate, caprylate, isobutyrate, oxalate, malonate, succinate, suberate, sebacate, fumarate, maleate, mandelate, benzoate, dinitro-benzoate, phthalate, benzenesulfonate, toluene-sulfonate, phenylacetate, citrate, lactate, tartrate, methanesulfonate, and the like. Also contemplated are salts of amino acids such as arginate and the like and gluconate, galacturonate and include acids related to the pharmaceutically acceptable salts listed in Journal of Pharmaceutical Science, 66, 2 (1977).

"C₁₋₁₂ alkyl" as used herein alone or in combination, refers to a straight or branched saturated hydrocarbon radical having 1 to 12 carbon atoms preferably 1 to 6 carbon atoms such as e.g. methyl, ethyl, n-propyl, isopropyl, n-butyl, secbutyl, isobutyl, ter-butyl, n-pentyl, 2-methylbutyl, 3-methylbutyl, n-hexyl, 4-methylpentyl, neopentyl, n-hexyl, 1,2-dimethylpropyl,2-2-dimethylpropyl and 1,2,2 trimethylpropyl. Alkyl moieties may optionally be substituted by 1 to 5 substituents selected from the group consisting of halogen, hydroxyl, cyano, azido, aryloxy, alkoxy, alkylthio, or aryl.

"Halogen" as used herein means fluorine, chlorine, bromine or iodine.

"C₂₋₁₂-alkenyl" as used herein refers to a straight or branched unsaturated hydrocarbon radicals or combinations thereof having 2-12 carbon atoms preferably 2-6 carbon atoms and one double bond such as e.g. vinyl, 1-propenyl, allyl, isopropenyl, n-butenyl, n-pentenyl and n-hexenyl. Alkenyl groups are optionally substituted with any suitable group including but not limited to one or more moieties selected from groups as described above for the alkyl groups.

"C₂₋₁₂-alkynyl" as used herein refers to a straight or branched unsaturated hydrocarbons or a combination thereof containing at least one carbon-carbon triple bond, such as e.g. -CcC-, -C≡CCH₂-, -CH₂C≡CCH₂-, - CH₂CH₂C≡C-,-CH₂CH₂C≡C CH₂CH₂-, -CH(CH₃)C≡CCH(CH₃)-, and the like.

Alkynyl groups are optionally substituted with any suitable group including but not limited to one or more moieties selected from groups as described above for the alkyl groups.

"Aryl" as used herein refers to mono or bicyclic aromatic rings having from 6 to 10 carbon atoms. Monocyclic aromatic ring, preferably have 6 members and bicyclic rings preferably have 8,9 or 10 membered ring structures. Diaryl (or diaralkyl) also refers to groups such as -CH₂-aryl-CH₂-aryl-CH₂-, - CH₂-aryl-aryl-CH₂-, -CH₂-aryl-CH=CH-aryl-CH₂, -CH₂-aryl-C≡C-aryl-CH₂-. Exemplary aryl groups include phenyl , naphthyl, o-xylyl, m-xylyl , p-xylyl, bis(methylene)-naphthyl and the like
wherein each aromatic ring is optionally substituted by 1 to 4 substituents.

"Cycloalkyl" as used herein alone or in combination refers to a monovalent group of 3 to 8 carbon atoms, usually 3 to 6 carbon atoms derived from a saturated or unsaturated cyclic hydrocarbon which may be substituted by any suitable group including but not limited to one or more moieties selected from the group as described above for the alkyl group. Preferred cycloalkyl groups are bis(methylene)-cycloalkanes or bis(methylene)-cycloalkenes.

The term bis(methylene)-cycloalkyl as used herein refers to bis(methylene)-cyclopropane, bis(methylene)-cyclobutane, bis(methylene)-cyclopentane, bis(methylene)-cyclohexane and the like.

The term bis(methylene)-cycloalkene as used herein refers to bis(methylene)-cyclopropene, bis(methylene)-cyclobutene, bis(methylene)-cyclopentene, bis(methylene)-cyclohexene and the like.

"Heterocycloalkyl" as used herein means a monocyclic alkyl group saturated or unsaturated having from 3 to 8 members including from 1 to 3 heteroatoms selected from N, O and S. Exemplary heterocycloalkyl include pyrrolidinyl, tetrahydrofuranyl, tetrahydrothienyl, piperidinyl, morpholinyl, piperazinyl, dioxanyl. In some preferred embodiments heterocycloalkyl may be substituted with 1 or 3 substituents. Preferred heterocycloalkyl groups are bis(methylene)-heterocycloalkanes or bis(methylene)-heterocycloalkenes.

"Heteroaryl" means 5 to 10 membered mono or bicyclic aromatic ring having from 1 to 3 heteroatoms selected from N, O and S. Monocyclic rings preferably have 5 or 6 members and bicyclic rings preferably have 8, 9 or 10 membered ring structures. Exemplary heteroaryls include pyrrolyl, furyl, thienyl, imidazolyl, pyrazolyl, oxazolyl, isoxazolyl, thiazolyl, isothiazolyl, pyridyl, pyrazinyl, pyrimidinyl, indolyl, quinolyl, isoquinolyl, quinoxalyl, benzodioxanyl. In some preferred embodiments heterocycloalkyl may be substituted with 1 or 3 susbtituents. Preferred heterocycloalkyl groups are bis(methylene)-heteroaryls.

Suitable substituents include, unless otherwise noted, halogen, cyano, alkoxy, alkylthio (and oxidized analogues such as sulfoxide and sulfone), C₁₋₃-alkyl, hydroxy, amino, amido, nitro, alkylamino, alkylamido, alkylsulfonamido, carboxyalkyl, carboxy, carbamide, dialkylamino, heterocycloalkyl, aryl, heteroaryl, perhaloalkyl or azido.

"Cyano" as used herein refers to a group of formula -CN.

"Hydroxy" as used herein refers to a group of formula -OH.

"Alkoxy" as used herein refers to a group of formula -OR' wherein R' is an alkyl as defined above.
"amido" as used herein refers to a group of formula -CONH₂
"amino" as used herein refers to a group of formula - NH₂
"nitro" as used herein refers to a group of formula -NO₂
"Azido" as used herein refers to a group of formula N₃.

"C₁₋₁₂-alkoxy" as used herein, alone or in combination, refers to a straight or branched monovalent substituent comprising a C₁₋₁₂-alkyl group preferably C₁₋₆-alkyl group linked through an ether oxygen having its free valence bond from the ether oxygen and having preferably 1 to 6 carbon atoms e.g. methoxy, ethoxy, propoxy, isopropoxy, butoxy, pentoxy and the like.

"C₁₋₁₂ alkylthio" as used herein alone or in combination, refers to a straight or branched saturated hydrocarbon chain having 1 to 12 carbon atoms preferably 1 to 5 carbon atoms linked to a sulphur atom such as e.g. methylthio, ethylthio, propylthio, butylthio, pentylthio.

"Arylthio" as used herein alone or in combination, refers to a group of formula - SR' wherein R' is an aryl as defined above.

" sulfoxide" as used herein refers to sulphur atom linked to one oxygen atom. "sulfone" as used herein refers to sulphur atom linked to two oxygen atoms.

"C₂₋₆ alkylenedioxy" as used herein refers to a bridge -O-(CH₂)n-O- with n equal 1 to 6 preferably 1 or 2 connecting two carbons in an unsaturated hydrocarbon chain or cycle having 2-6 carbon atoms and at least one double bond

"Ether" as used herein alone or in combination refers to a group of formula R'-O-R' wherein the oxygen atom may form part of a chain or a ring
and R', which may be the same or different, refers to an alkyl or an alkenyl as defined above.
Preferred ether group are e.g. -(CH₂)n-O-(CH₂)n- with n equals 1 to 5, -(CH₂)-O-(CH₂)n -O-(CH₂)- with n equals 2 to 4 , and the like.

"Thioether" as used herein alone or in combination refers to a group of formula R'-S-R' wherein the sulphur atom may form part of a chain or a ring
and R', which may be the same or different, refers to an alkyl or an alkenyl as defined above.
Preferred thioether group are e.g. -(CH₂)n-S-(CH₂)n- with n equals 1 to 5, - (CH₂)-S-(CH₂)n -S-(CH₂)- with n equals 2 to 4 , and the like.

"Urea" as used herein alone or in combination refers to -NH-CO-NH-.
The term "thiourea" as used herein alone or in combination refers to -NH-CS-NH-. Preferred urea groups are N,N'-(alkylene)ureas. Alkylene means -(CH₂)n-with n an integer from 1 to 3.

"Guanidine" as used herein refers to a group of formula -NHC(=NX')NH-- wherein X' is selected from the group consisting of hydrogen, alkyl, or aryl as defined above. Preferred guanidine groups are N,N'-(alkylene)guanidines. Alkylene means -(CH₂)n- with n an integer from 1 to 3

"Perhaloalkyl" as used herein refers to a straight or branched saturated or unsaturated hydrocarbon chain having 1 to 12 carbon atoms preferably 1 to 6 carbon atoms substituted by halogen instead of hydrogen such as trifluoromethyl group.

In a particular aspect, the present invention relates to the use of bis-1,2,3,4-tetrahydroisoquinoline derivatives of formula (I), any stereoisomer thereof, including optical isomer or mixture of optical isomers thereof, including racemic mixture or any tautomeric or polymorphic form thereof and their pharmaceutically acceptable salt thereof 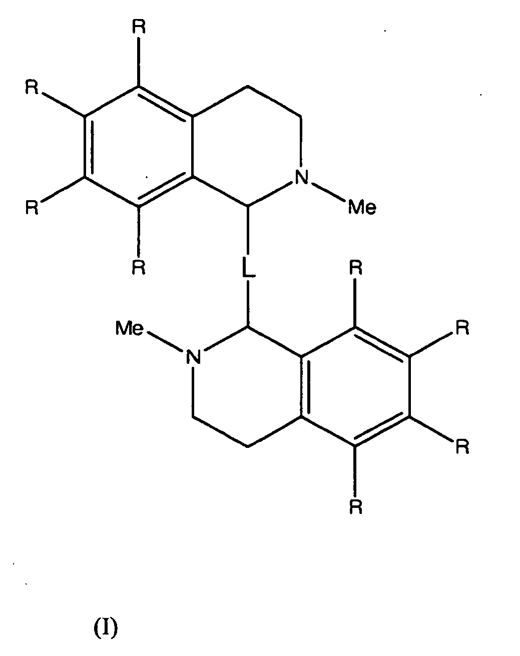 wherein
R represents hydrogen, hydroxy, C₁₋₆ alkyl, C₁₋₆ alkoxy or a halogen and
L represents C₁₋₆alkyl, C₂₋₆alkenyl, a C₂₋₆alkynyl, an aryl, a bis (methylene)-cycloalkyl or a bis(methylene)cycloalkene;
as SK channel blockers.

Such derivatives possess a configuration particularly well adapted to interact with Apamin-sensitive sites of SK channels thereby leading to an inhibition of the after hypolarization of the neuron because of blockade of SK channels.

Preferably, the present invention relates to bis 1,2,3,4-tetrahydroisoquinoline derivatives of formula (I) wherein
R represents C₁₋₆₋alkyl or C₁₋₆alkylthio; and
L represents C₁₋₆alkyl, C₂₋₆₋alkenyl, a C₂₋₆₋alkynyl, an aryl, a bis (methylene)-cycloalkyl or a bis(methylene)cycloalkene

In a further particular aspect, the present invention relates to the use of bis 1,2,3,4-tetrahydroisoquinoline derivatives of formula (I) selected from the group consisting of:
*1,3-Bis[1-(2-methy*/*-1,2,3,4-tetrahydrolisoquinolyl)]-properne hydrochloride 4a*
*1,4-Bis[1-(2-methyl-1,2,3,4-tetrahydroisoquinoly*/*)]-butane hydrochloride 4b*
*1,5-Bis[1-(2-methyl-1,2,3,4-tetrahydroisoquinolyl)]-pentane hydrochloride 4c*
*1,3-Bis[1-(6,7-dimethoxy-2-methyl-1,2,3,4-tetrahydroisoquinolyl)]-propane hydrochloride 4d*
*1,4-Bis[1-(6,7-dimethoxy-2-methyl-1,2,3,4-tetrahydroisoquinolyl)]-butane hydrochloride 4e*
*1,5-Bis[1-(6,7-dimethoxy-2-melhyl-1,2,3,4-telrahydroisoquinolyl)]-pentane hydrochloride 4f*
*1,3-Bis[1-(6,7,8-trimethoxy-2-methyl-1,2,3,4-tetrahydroisoquinolyl)]-propane hydrochloride 4g*
*1,4-Bis[1-(6,7,8-trimethoy-2-methyl-1,2,3,4-tetrahydroisoquinolyl)]-butane hydrochloride 4h*
*o-Bis[1-(2-methyl-1,2,3,4-tetrahydroisoquinolyl)]-xylene hydrochloride 4j*
*m-Bis[1-(2-methyl-1,2,3,4-tetrahydroisoquinolyl)]-xylene hydrochloride 4k*
*p-Bis[1-(2-methyl-1,2,3,4-tetrahydroisoquinolyl)]-xylene hydrochloride 41*
*o-Bis[1-(6,7-dimethoy--2-methyl-1,2,3,4-tetrahydroisoquinolyl)]-xylene hydrochloride 4m*
*m-Bis[1-(6,7-dimethoxy-2-methyl-1,2,3,4-tetrahydroisoquinolyl)]-xylene hydrochloride 4n*
*p-Bis[1-(6,7-dimethoxy-2-methyl-1,2,3,4-tetrahydroisoquinolyl)]-xylene4o*
*o-Bis[1-(6,7,8-trimethoxy-2-methyl-1,2,3,4-tetrahydroisoquinolyl)]-xylene hydrochloride 4p*
*m-Bis[1-(6,7,8-trimethoxy-2-methyl-1,2,3,4-tetrahydroisoquinolyl)]-xylene hydrochloride 4q*
*p-Bis[1-(6,7,8-trimethoxy-2-methyl- 1,2,3,4-tetrahydroisoquinolyl)]-xylene 4r*
*1,3-bis[1-(2,8-dimethyl-1,2,3,4-tetrahydroisoquinolyl)]-propane hydrochloride 4s*
*1,3-bis[1-(5-bromo-2-methyl-1,2,3,4-tetrahyroisoquinolyl)]-propane hydrochloride 4t*
*1,1'-bis[1-(2-methyl-1,2,3,4-tetrahydroisoquinolyl)]-dimethylether 4u*
as SK channel blockers.

The present invention also relates to a pharmaceutical composition comprising an effective amount of bis 1,2,3,4-tetrahydroisoquinoline derivatives of formula (I) or a pharmaceutical acceptable salt thereof with a pharmaceutical acceptable acid or base, or any stereoisomer such as an optical isomer or mixture of optical isomer, including racemic mixture or any tautomeric form together with one or more acceptable carriers or diluents.

The present invention further relates to compounds of formula (I) for use as a medicament.The invention further relates to a method of treatment of diseases of the central nervous system in mammals preferably a human suffering from such diseases comprising administration of a therapeutically effective amount of a composition containing a bis-1,2,3,4-tetrahydroisoquinoline derivatives of formula (I).

In a particular embodiment, the present invention relates to the use of compounds of formula (I) for the manufacture of a medicament for the treatment of diseases of the central nervous system.

By diseases of central nervous system, one means for example Alzheimer's disease, Parkinson's disease and other neurodegenerative disorders, schizophrenia and other psychotic disorders, cognitive dysfunction, alcohol and drug addiction, depression and the like. Such diseases of central nervous system are described in Harrisson's Principles of Internal Medicines 14th ed Mc Graw-Hill

Thus in a further particular embodiment, the present invention relates to the use of compounds of formula (I) for the manufacture of a medicament for the treatment or prevention of Alzheimer's disease, Parkinson's disease, and other neurodegenerative disorders, schizophrenia, and other psychotic disorders, cognitive dysfunction, alcohol and drug addiction, and depression.

By method of treatment, one means prevention, amelioration or reduction in severity of a symptom or a combination of symptoms of a disease of the central nervous system

Bis(1,2,3,4)-tetrahydroisoquinoline derivatives may be administered orally, rectally, parenterally or topically to the skin and mucosa.

Examples of the form of pharmaceutical composition comprising a bis 1,2,3,4-tetrahydroisoquinoline derivatives of formula (I), include tablets, capsules, solutions, emulsions, suspensions, powders and granules. They may be produced through well-known techniques by use of additives such as excipients, lubricants and binders. Typically bis 1,2,3,4-tetrahydroisoquinoline derivatives of the invention are administered in an amount of about 0.01 to 1000 mg/kg and preferably 0.5 to 500 mg/kg once or twice daily. However other amounts including substantially lower or higher amounts, may also be administered. The bis 1,2,3,4-tetrahydroisoquinoline derivatives of the present invention are administered to a mammal preferably a human subject in need of treatment intramusculary, subcutaneously, intravenously, by any other acceptable route of administration.

Different amounts of the bis 1,2,3,4- tetrahydroisoquinoline derivatives of formula (I) may also be administered as seen suitable by a practitioner for specific cases. For this or any other application the bis 1,2,3,4-tetrahydroisoquinoline derivatives of the present invention may be administered in an amount of about 0.01 to 1000 mg/kg and more preferably 0.5 to 500 mg/kg. Any means of administration is suitable. The foregoing ranges are however suggestive, as the number of variables in regard to an individual treatment regime is large, and considerable variations from these recommended values are expected.

In order to administer therapeutic agents based on, or derived from, the present invention, it will be appreciated that suitable carriers, excipients, and other agents may be incorporated into the compositions to provide improved transfer, delivery, tolerance, and the like.

A multitude of appropriate compositions can be found in the well known formulary entitled: Remington's Pharmaceutical Sciences, (15th Edition, Mack Publishing Company, Easton, Pennsylvania (1975)), particularly Chapter 87, by Blaug, Seymour, therein. These formulations include for example, powders, pastes, ointments, jellies, waxes, oils, lipids, anhydrous absorption bases, oil-in-water or water-in-oil emulsions, emulsions carbowax (polyethylene glycols of a variety of molecular weights), semi-solid gels, and semi-solid mixtures containing carbowax.

Any of the foregoing compositions may be appropriate in treatments and therapies in accordance with the present invention, provided that the active agent in the formulation is not inactivated by the composition and the composition is physiologically compatible.

The quantities of active ingredient necessary for effective therapy will depend on many different factors, including means of administration, target site, physiological state of the patient, and other medications administered. Thus, treatment dosages should be titrated to optimize safety and efficacy. Typically, dosages used *in vitro* may provide useful guidance in determining the amounts useful for *in situ* administration of the active ingredients. Animal testing of effective doses for treatment of particular disorders will provide further predictive indication of human dosage. Various considerations are described, for example, in Goodman and Gilman's the Pharmacological Basis of Therapeutics, 7th Edition (1985), MacMillan Publishing Company, New York, and Remington's Pharmaceutical Sciences 18th Edition, (1990) Mack Publishing Co, Easton Penn. Methods for administration are discussed therein, including oral, intravenous, intraperitoneal, intramuscular, transdermal, nasal, iontophoretic administration, and the like.

The compositions of the invention may be administered in a variety of unit dosage forms depending on the method of administration. For example, unit dosage forms suitable for oral administration include solid dosage forms such as powder, tablets, pills, capsules, and dragees, and liquid dosage forms, such as elixirs, syrups, and suspensions. The active ingredients may also be administered parenterally in sterile liquid dosage forms. Gelatin capsules contain the active ingredient and as inactive ingredients powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The concentration of the compositions of the invention in the pharmaceutical formulations can vary widely, i.e., from less than about 0.1%, usually or at least about 2% to as much as 20% to 50% or more by weight, and will be selected primarily by fluid volumes, viscosities, etc., in accordance with the particular mode of administration selected.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention, and more preferably at a concentration of 25%-75%.

A preferred pharmaceutical composition comprises :
Active compound : 5.0 mg
Lactosum : 70 mg
Avicel ® : 30 mg
Magnesium stearas 0.25 mg
is prepared by conventional tabletting techniques.

The method of production of the bis 1,2,3,4-tetrahydroisoquinoline derivatives of formula (I) is illustrated in scheme 1.

### Scheme 1 : General Chemical Pathways

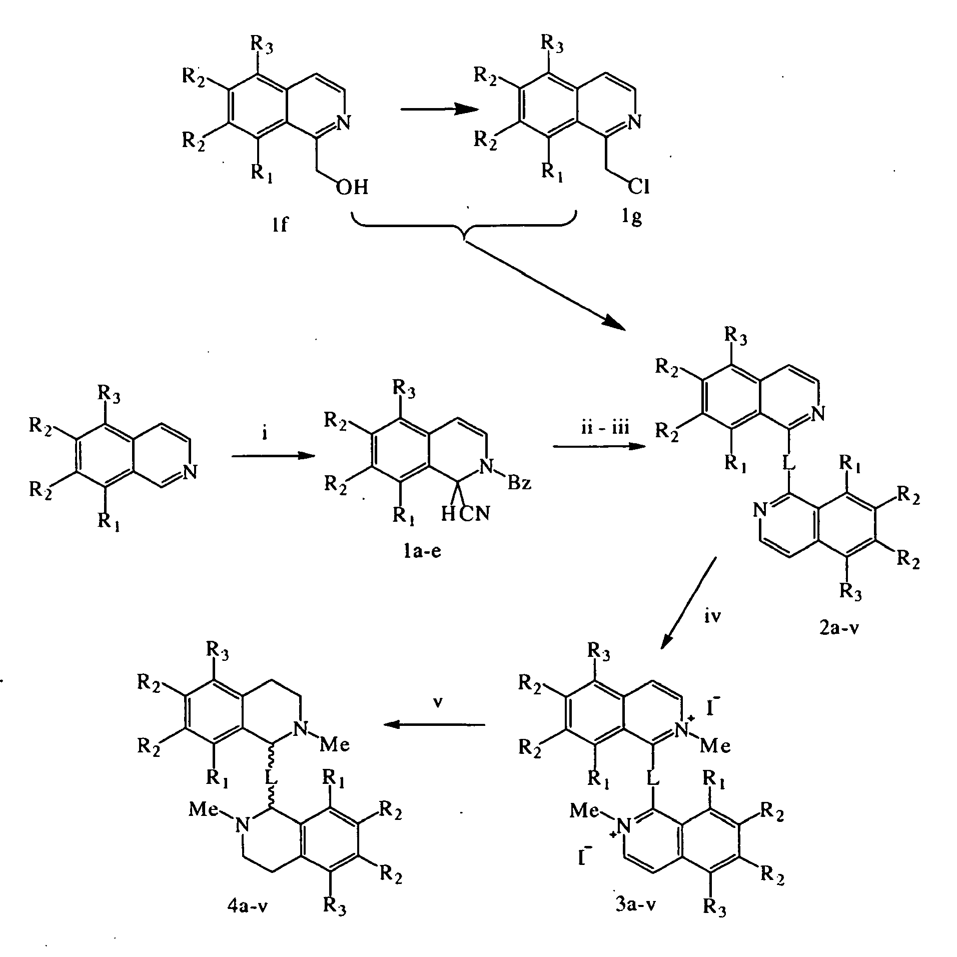

wherein i: Me₃SiCN, BzCl, AlCl₃, CH₂Cl₂, rt;
ii: X-L-X (X = Br or I), NaH, DMF, -10 °C;
iii: NaOH, EtOH/ H₂O, under reflux;
iv: MeI, DMF, Δt until dissolution;
v: NaBH₄, MeOH, at room temperature

### Chemistry

The syntheses of methoxyisoquinolines were classically carried out by using a modification of the Pomeranz-Fritsch synthesis. Then the dimerisation was performed by using the Reissert compound pathway (Scheme 1). The Reissert compounds (la-e) were obtained by reaction of the corresponding isoquinoline with benzoyl chloride in the presence of trimethylsilyl cyanide. This reaction was carried out in CH₂Cl₂ and gave the Reissert compounds in good yield. Then these derivatives were deprotonated by sodium hydride in DMF. The resulting Reissert anions were alkylated by using a half equivalent of the appropriate biselectrophilic reagent. Then the alkylated Reissert compounds were hydrolysed to bis-isoquinolines (2a-t). 2v is prepared from 1-hydroxymethyl-isoquinoline (1f) and 1-chloromethyl-isoquinoline (1g) in basic conditions following a nucleophilic substitution of the chlorine in DMF. The dimeric isoquinolines were methylated by methyl iodide in DMF under mild warming to obtain bis-isoquinolinium derivatives (3a-v). Finally these bis-isoquinolinium derivatives (3a-v) were reduced by using NaBH₄ in order to obtain the bis 1,2,3,4-tetrahydroisoquinoline derivatives (4a-r). These compounds (4a-v) were screened in binding tests and the most active derivatives (4d-h and m-n) were separated by using a Chiralcel OD-H column. All separated isomers were then tested in order to determine their affinity for apamin sensitive sites.

The method of production is also illustrated by the hereafter examples:

### Experimental Sections

### 1. Chemistry

Melting points were determined on a Buchi-Tottoli capillary melting point apparatus in open capillary and are uncorrected. NMR spectra were recorded on a Bruker Avance 500 spectrometer at 500 MHz. IR spectra were measured using KBr discs on a Perkin-Elmer FTIR-1750 spectrometer. Only significant bands from IR are reported. Elemental analyses were determined using a Carlo-Erba elemental analyser CHNS-O model EA1108 and the results are within 0.4 % of the theorical values. Mass spectra were recorded on a QTOF II (Micromass, Manchester UK) spectrometer with electrospray mode. All starting materials and reagents were obtained from Aldrich Chemical Co. and were used without further purification. Separations by column chromatography were carried out using Merck Kieselgel 60 (230-400 mesh). Concentration and evaporation refer to removal of volatile materials under reduced pressure (10-15 mm Hg at 30-50 °C) on a Buchi Rotavapor.
The methoxylated isoquinolines were prepared by the Jackson modification of the Pomeranz-Fritsch synthesis [Birch et al, J. Chem. Soc. Perkin I, 1974, 2185-2190]. 2-Benzoyl-1-cyano-1,2-dihydroisoquinoline *1a* was obtained from isoquinoline by the procedure described by Uff et al.[Uff et al, Org. Syn., 1977, 56, 19-25].

### 2. Chiral separations

Chiral separations were performed on a HPLC column Chiralcel^{®}) OD-H 20 x 250 mm using n-hexane/isopropanol or MeCN as mobile phase. Diethylamine was used as organic modifier. After separations of compounds 4d-h and 4m-n, the collected fractions were evaporated under reduced pressure and then kept three days under vacuum and dry atmosphere. Then the compounds were dissolved in Et₂O and the corresponding hydrochlorides were precipitated by the addition of an etherous HCl solution. The precipitate were collected, washed with Et₂O and kept under dry atmosphere until testing.

### Preparation of 2-benzoyl-1-cyano-6,7-dimerhoxy-1,2-dihydroisoquinoline 1b

Anhydrous aluminum chloride (10 mg) was added to a stirred solution of 6,7-dimethoxyisoquinoline (2.97 g ; 15.7 mmol) and trimethylsilyl cyanide (3.9 mL ; 31.4 mmol) in anhydrous CH₂Cl₂ (50 mL) at room temperature. Then benzoyl chloride (3.6 mL ; 31.4 mmol) was dropwise added to the stirred solution over a course of 5 min. The mixture was warmed to 30 °C if no exotherm has began after the addition of benzoyl chloride. After stirring for a further 3 h period, water (50 mL) was added and stirred continuously for 30 min. The organic layer was collected and washed successively with 1N aqueous HCl (2 x 50 mL), water (50 mL), 1 N aqueous NaOH (2 x 50 mL) and finally water (50 mL). The organic solution was dried over anhydrous MgSO₄ and evapored under reduced pressure to give an oil which was triturated with Et₂O (20 mL) resulting in cristallisation. The solid was collected, washed with small volumes of Et₂O and dried (3.5 g) ; *yield,* 70 % ; *mp* 155-157 °C
*¹H-NMR* (CDCl₃) δ 3.92 (s, 3H), 3.94 (s, 3H), 5.99 (br d, 1H, *J = 6.6 Hz*), 6.51 (br s, 2H), 6.72 (s, 1H), 6.85 (br s, 1H), 7.47 (t, 2H, *J* = *7.4 Hz*), 7.55 (t, 1H, *J* = *7.4 Hz*) 7.60 (d, 2H, *J* = 7.4 *Hz*)
*Anal.* C₁₉H₁₆N₂O₃ (320.348) Calc. N 8.74 C 71.24 H 5.03 Found N 8.73 C 71.19 H 4.85

### Preparation of 2-benzoyl-1-cyano-6,7,8-trimethoxy-1,2-dihydroisoquinoline 1c

Compound 1c was prepared according to the same chemical procedure as described for compound 1b using 6,7,8-trimethoxyisoquinoline as starting material ; *yield,* 60 % ; *mp* 158-160 °C
*¹H-NMR* (CDCl₃) δ 3.89 (s, 3H), 3.90 (s, 3H), 4.08 (s, 3H), 5.90 (br d, 1H, *J* = *6.2 Hz*), 6.49 (s, 1H), 6.56 (br s, 1H), 6.75 (br s, 1H), 7.47 (t, 2H, *J = 7.5 Hz*), 7.55 (t, 1H, *J* = *7.5 Hz*) 7.60 (d, 2H, *J* = *7.5 Hz*)
*Anal.* C₂₀H₁₈N₂O₄ (350.374) Calc. N 8.00 C 68.56 H 5.18 Found N 8.08 C 68.49 H 5.12

### Preparation of 2-benzoyl-1-cyano-8-methyl-1,2-dihydroisoquinoline 1d

Compound 1d was prepared according to the same chemical procedure as described for compound 1b using 8-methylisoquinoline as starting material ; *yield,* 85 % ; *mp* 149-151°C
*¹H-NMR* (CDCl₃) δ 2.48 (s, 3H), 6.01 (d, 1H, *J = 7.3 Hz*), 6.58 (br s, 1H), 6.69 (br s, 1H), 7.03 (d, 1H, *J* = *7.6 Hz*), 7.16 (d, 1H, *J* = *7.6 Hz*), 7.27 (t, 1H, *J* = *7.6 Hz*) 7.45 (t, 2H, *J* = *7.6 Hz*), 7.54 (t, 1H(d, 1H, *J = 7.6 Hz*), 7.59 (d, 2H(d, 1H, *J* = *7.6 Hz*)
*Anal.* C₁₈H₁₄N₂O (274.323) Calc. N 10.21 C 78.81 H 5.14 Found N 10.61 C 78.85 H 5.39

### Preparation of 2-benzoyl-5-bromo-1-cycrno-1,2-dihydroisoquinoline 1e

Compound 1e was prepared according to the same chemical procedure as described for compound 1b using 5-bromoisoquinoline as starting material ; *yield*, 87 % ; *mp* 177-178°C
*¹H-NMR* (CDCl₃) δ 6.42 (d, 1H, *J* = *7.8 Hz*)*,* 6.55 (br s, 1H), 6.74 (br d, 1H, *J* = *7.8 Hz*), 7.21 (t, 1H, *J* = *7.7 Hz*), 7.31 (d, 1H, *J* = *7.7 Hz*), 7.50 (t, 2H, *J* = *7.5 Hz*) 7.57-7.65 (m, 4H)
*Anal.* C₁₇H₁₁N₂OBr (339.198) Calc. N 8.26 C 60.20 H 3.27 Found N 8.19 C 60.13 H 3.27

### Preparation of 1-hydroxymethyl-isoquinoline 1f

A solution of 2-benzoyl-1-cyano-1,2-dihydroisoquinoline 1a (5.0 g ; 19.2 mmol) in DMF (15 mL) was dropwise added to a stirred suspension of sodium hydride (0.46 g ; 19.2 mmol) and paraformaldehyde (0.62 g ; 19.2 mmol) in DMF (15 mL) at - 10 °C. The content was stirred for 1 h and poured into ice-cold water (200 mL). The aqueous solution was extracted with CH₂Cl₂ (3 x 50 ml). The organic layers were dried over anhydrous MgSO₄ and evaporated under reduced pressure to afford oil. This oil was dissolved in EtOH and refluxed with aqueous NaOH (2g in 2 mL H₂O) during 15 min. After removal of EtOH, the crude residue was dissolved in water (150 mL). The aqueous solution was extracted with CH₂Cl₂ (3 x 30 mL). The organic layers were dried over anhydrous MgSO₄ and evaporated under reduced pressure to afford a cream solid which recrystallized from Et₂O/n-hexane ; *yield*, 88 %
¹H-NMR (DMSO) δ 5.50 (s, 2H), 7.98 (t, 1H, *J* = *7.6 Hz*), 8.18 (t, 1H, *J* = *7.6 Hz*), 8.33 (d, 1H, *J* = *8.2 Hz*), 8.37 (d, 1H, *J* = *6.5 Hz*), 8.48 (d, 1H, *J* = *6.5 Hz*), 8.53 (d, 1H, *J* = *8.5 Hz*)

### Preparation of 1-chloromethy/-isoquinoline 1g

An excess of thionyl chloride (1.8 mL ; 25 mmol) was dropwise added to a stirred ice-cold solution of 1-hydroxymethyl-isoquinoline 1f (2.0 g ; 12.6 mmol) in CHCl₃ during 30 min. The reaction medium was poured into an ice (100g)-water (50 mL)-NH₄OH (10 mL) mixture and then stirred during 20 min. The organic layer was collected, dried over anhydrous MgSO₄ and evaporated under reduced pressure. The resulting oil was purified by flash chromatography (Me₂CO) to afford a white solid (2 g); *yield,* 90 %
¹H-NMR (CDCl₃) δ 5.18 (s, 2H), 7.68-7.76 (m, 3H), 7.88 (d, 1H, *J* = *8.1 Hz*), 8_{.}27 (d, 1H, *J* = *7.8 Hz*), 8.49 (d, 1H, *J* = *5.7 Hz*)

### Preparation of 1,3-bis(1-isoquinolyl)-propane 2a

A solution of 2-benzoyl-1-cyano-1,2-dihydroisoquinoline 1a (5.0 g ; 19.2 mmol) and of 1,3-diiodopropane (1.1 mL ; 9.6 mmol) in DMF (15 mL) was dropwise added to a stirred suspension of sodium hydride (0.46 g; 19.2 mmol) in DMF (30 mL) at - 10 °C. The content was stirred for 4 h and poured into ice-cold water (200 mL). The creamy solid was filtered off. After drying, the solid was hydrolyzed by treatment with 50 % aqueous NaOH in EtOH at reflux. After removal of EtOH, the crude residue was dissolved in ArMe (50 mL) and water (50 mL). The organic layer was collected, washed with water (50 mL) and then extracted with 1N aqueous HCl (2 x 50 mL). The acidic layers were basified with concentrated NH₄OH and finally extracted with CH₂Cl₂ (3 x 30 mL). The organic layers were dried over anhydrous MgSO₄ and evaporated under reduced pressure to afford a white solid which recrystallized from petroleum ether 100-140 (2.5 g) ; *yield*, 40 % ; *mp* 96-97 °C
*¹H-NMR* (CDCl₃) δ 2.49 (pentuplet, 2H, *J* = 7.7 *Hz),* 3.50 (t, 4H, *J* = *7.7 Hz*), 7.51 (d, 2H, *J* = *5.7 Hz*), 7.56 (t, 2H, *J* = *7.5 Hz*), 7.65 (t, 2H, *J* = *7.5 Hz*), 7.80 (d, 2H, *J* = *7.5 Hz*), 8.18 (d, 2H, *J* = *7.5 Hz),* 8.44 (d, 2H, *J* = *5.7 Hz*)
*Anal.* C₂₁H₁₈N₂ (298.389) Calc. N 9.39 C 84.53 H 6.08 Found N 9.56 C 84.38 H 6.16

### Preparation of 1,4-bis(1-isoquinolyl)-butane 2b

Compound 2b was prepared according to the same chemical procedure as described for compound 2a using compound 1a as starting material and using 1,4-dibromobutane instead of 1,3-diiodopropane. The resulting solid was recrystallized from ArMe/n-hexane ; *yield* 33 % *mp* 120-121 °C
*¹H-NMR* (CDCl₃) δ 2.07 (t, 4H, *J* = *7.4 Hz),* 3.40 (t, 4H, *J* = *7.4 Hz),* 7.50 (d, 2H, *J* = *5.7 Hz*), 7.58 (t, 2H, *J* = *7.5 Hz*), 7.66 (t, 2H, *J* = *7.5 Hz*), 7.80 (d, 2H, *J* = *8.1 Hz*), 8.17 (d, 2H, *J* = *8.4 Hz*), 8.43 (d, 2H, *J* = *5.7 Hz*)
*Anal.* C₂₂H₂₀N₂ (312.416) Calc. N 8.97 C 84.58 H 6.45 Found N 8.91 C 84.23 H 6.32

### Preparation of 1,5-bis(1-isoquinolyl)-pentane hydrochloride 2c

Compound 2c was prepared according to the same chemical procedure as described for compound 2a using compound 1a as starting material and using 1,5-dibromopentane instead of 1,3-diiodopropane. The compound was isolated as hydrochloride salt and further recrystallized from MeCN ; *yield,* 30 % ; *mp* 202-204 °C
*¹H-NMR* (DMSO) δ 1.59 (m, 2H, *J* = *7.5 Hz),* 1.90 (m, 4H, *J* = *7.5 Hz),* 3.62 (t, 4H, *J* = *7.5 Hz*), 7.98 (t, 2H, *J* = *7.6 Hz),* 8.17 (t, 2H, *J* = *7.6 Hz*), 8.30 (d, 2H, *J* = *8.3 Hz*), 8.32 (d, 2H, *J* = *6.5 Hz*), 8.51 (d, 2H, *J* = *6.5 Hz),* 8.66 (d, 2H, *J* = *8.5 Hz*)
*Anal.* C₂₃H₂₄N₂Cl₂ (417.374) Calc. N 6.71 C 66.19 H 6.28 Found N 6.61 C 65.84 H 5.91

### Preparation of 1,3-bis[1-(6,7- dimethoxyisoquinolyl)]-propane 2d

Compound 2d was prepared according to the same chemical procedure as described for compound 2a using compound 1b as starting material. But after alkaline hydrolysis and evaporation of EtOH the residue was dispersed in water (100 mL). The precipitate was filtered, dried and recrystallized from ArMe ; *yield,* 31 % ; *mp* 182-183 °C
*¹H-NMR* (CDCl₃) δ 2.49 (pentuplet, 2H, *J* = *7.4 Hz),* 3.38 (t, 4H, *J = 7.4 Hz*), 3.90 (s, 6H), 4.01 (s, 6H), 7.04 (s, 2H), 7.28 (s, 2H), 7.38 (d, 2H, *J* = *5.6 Hz*), 8.31 (d, 2H, *J = 5.6 Hz*)
*Anal.* C₂₅H₂₆N₂O₄ (418.493) Calc. N 6.69 C 71.75 H 6.26 Found N 6.74 C 71.97 H 6.28

### Preparation of 1,4-bis[1-(6,7-dimethoxyisoquinolyl)]-butane 2e

Compound 2e was prepared according to the same chemical procedure as described for compound 2a using compound 1b as starting material and using 1,4-dibromobutane instead of 1,3-diiodopropane. But after alkaline hydrolysis and evaporation of EtOH the residue was dispersed in water (100 mL). The precipitate was filtered, dried and recrystallized from ArMe ; *yield,* 39 % ; *mp* 186-188 °C
*¹H-NMR* (CDCl₃) δ 2.08 (br s, 4H), 3.31 (br s, 4H), 3.99 (s, 6H), 4.02 (s, 6H), 7.04 (s, 2H), 7.31 (s, 2H), 7.36 (d, 2H, *J = 5.6 Hz*), 8.30 (d, 2H, *J = 5.6 Hz*)
*Anal.* C₂₆H₂₈N₂O₄ (432.520) Calc. N 6.48 C 72.20 H 6.53 Found N 6.47 C 71.88 H 6.67

### Preparation of 1,5-bis[1-(6,7-dimethoxyisoquinolyl)]-pentane 2f

Compound 2f was prepared according to the same chemical procedure as described for compound 2a using compound 1b as starting material and using 1,5-dibromopentane instead of 1,3-diiodopropane. But after alkaline hydrolysis and evaporation of EtOH the residue was dispersed in water (100 mL). The precipitate was filtered, dried and recrystallized from ArMe/n-hexane ; *yield,* 37 % ; *mp* 148-150 °C
*¹H-NMR* (CDCl₃) δ 1.66 (pentuplet, 2H, *J* = *7.5 Hz),* 1.97 (pentuplet, 4H, *J* = *7.5 Hz*), 3.23 (t, 4H, *J* = *7.5 Hz*), 3.99 (s, 6H), 4.02 (s, 6H), 7.05 (s, 2H), 7.30 (s, 2H), 7.36 (d, 2H, *J* = *5.6 Hz),* 8.30 (d, 2H, *J = 5.6 Hz*)
*Anal.* C₂₇H₃₀N₂O₄ (446.547) Calc. N 6.27 C 72.62 H 6.77 Found N 6.28 C 72.62 H 7.13

### Preparation of 1,3-bis[1-(6,7,8-trimethoxyisoquinolyl)]-propane hydrochloride 2g

Compound 2g was prepared according to the same chemical procedure as described for compound 2a using compound 1c as starting material. The resulting oil was purified by flash chromatography (Me₂CO). The oil was then isolated as hydrochloride salt and further recrystallized from EtOH/Et₂O ; *yield*, 38 % ; *mp* 181-182 °C dec.
*¹H-NMR* (CDCl₃) δ 2.50 (pentuplet, 2H, *J* = *7.3 Hz),* 3.99-4.02 (m, 10H), 4.08 (s, 6H), 4.36 (s, 6H), 7.03 (s, 2H), 7.72 (d, 2H, *J* = *6.5 Hz*), 8.12 (br t, 2H)
*Anal.* C₂₇H₃₂N₂O₆Cl₂ (551.461) Calc. N 5.08 C 58.81 H 5.85 Found N 4.96 C 58.47 H 6.04

### Preparation of 1,4-bis-[1-(6,7,8-trimethoyisoquinolyl)]-butane 2h

Compound 2h was prepared according to the same chemical procedure as described for compound 2a using compound 1c as starting material and using 1,4-diiodobutane instead of 1,3-diiodopropane. The crude residue was purified by flash chromatography (Me₂CO).The resulting solid was recrystallized from McCOOEt; *yield*, 13 % ; *mp* 156-158 °C dec.
*¹H-NMR* (CDCl₃) δ 1.97 (br m, 4H), 3.47 (br t, 4H), 3.93 (s, 6H), 3.99 (s, 6H), 4.01 (s, 6H), 6.86 (s, 2H), 7.30 (d, 2H, *J* = *5.6 Hz*), 8.27 (d, 2H, *J* = *5.6 Hz*) *Anal.* C₂₈H₃₂N₂O₆ (492.572) Calc. N 5.69 C 68.28 H 6.55 Found N 5.74 C 68.52 H 6.60

### Preparation of 1,5-bis[1-(6,7,8-trimethoxyisoquinolyl)]-pentane hydrochloride 2i

Compound 2i was prepared according to the same chemical procedure as described for compound 2a using compound 1c as starting material and using 1,5-diodopentane instead of 1,3-diiodopropane. The compound was isolated as hydrochloride salt and further recrystallized from EtOH/Et₂O ; *yield,* 3 %
*¹H-NMR* (DMSO) δ 1.62 (br m, 2H, *J = 7.6 Hz),* 1.81 (br m, 4H, *J = 7.6 Hz),* 3.56 (br t, 4H, *J = 7.6 Hz*), 3.90 (s, 6H), 4.06 (s, 6H), 4.08 (s, 6H), 7.61 (s, 2H), 8.09 (d, 2H, *J = 6.5 Hz*), 8.29 (d, 2H, *J = 6.5 Hz*), 15.87 (br s, 2H)

### Preparation of o-bis(1-isoquinolyl)-xylene 2j

Compound 2j was prepared according to the same chemical procedure as described for compound 2a using compound 1a as starting material and using α,α'-dibromo-o-xylene instead of 1,3-diiodopropane. The crude residue was purified by flash chromatography (MeCOOEt). The resulting solid was recrystallized from MeCOOEt ; *yield*, 38 % ; *mp* 146-148 °C
*¹H-NMR* (CDCl₃) δ 4.80 (s, 4H), 6.91 (dd, 2H, *J* = *3.4 and 5.6 Hz),* 7.06 (dd, 2H, *J = 3.4 and 5.6 Hz*), 7.51 (t, 2H, *J* = *7.6 Hz*), 7.54 (d, 2H, *J* = *5.7 Hz*), 7.64 (t, 2H, *J* = *7.6 Hz*), 7.82 (d, 2H, *J* = *8.2 Hz*), 8.11 (d, 2H, *J* = *8.5 Hz*), 8.47 (d, 2H, *J* = *5.7 Hz*)
*Anal.* C₂₆H₂₀N₂ (360.460) Calc. N 7.77 C 86.64 H 5.59 Found N 7.90 C 86.59 H 5.72

### Preparation of m-bis(1-isoquinolyl)-xylene 2k

Compound 2k was prepared according to the same chemical procedure as described for compound 2a using compound 1a as starting material and using α,α'-dibromo-m-xylene instead of 1,3-diiodopropane. The resulting solid was recrystallized from MeCOOEt ; *yield,* 36 % ; *mp* 114-116 °C
*¹H-NMR* (CDCl₃) δ 4.60 (s, 4H), 7.05 (d, 2H, *J* = *7.2 Hz*), 7.11 (t, 1H, *J* = *7.2 Hz*), 7.23 (s, 1H), 7.43 (t, 2H, *J = 7.6 Hz*), 7.54 (d, 2H, *J* = *5.7Hz*), 7.61 (t, 2H, *J = 7.6 Hz*), 7.79 (d, 2H, *J* = *8.2 Hz*), 8.04 (d, 2H, *J = 8.5 Hz*), 8.47 (d, 2H, *J* = *5.7 Hz*)
*Anal.* C₂₆H₂₀N₂ (360.460) Calc. N 7.77 C 86.64 H 5.59 Found N 7.89 C 86.65 H 5.71

### Preparation of p-bis(1-isoquinolyl)-xylene 21

Compound 21 was prepared according to the same chemical procedure as described for compound 2a using compound 1a as starting material and using α,α'-dibromo-p-xylene instead of 1,3-diiodopropane. But after alkaline hydrolysis and evaporation of EtOH the residue was dispersed in water (100 mL). The precipitate was filtered, dried and recrystallized from ArMe/n-hexane ; *yield*, 41 %; *mp* 183-185 °C
*¹H-NMR* (CDCl₃) δ 4.60 (s, 4H), 7.16 (s, 4H), 7.49 (t, 2H, *J* = *8.1 Hz),* 7.53 (d, 2H, *J* = *5.7 Hz*), 7.60 (t, 2H, *J* = *8.1 Hz*), 7.78 (d, 2H, *J* = *8.1 Hz*), 8.11 (d, 2H, *J* = *8.1 Nz*), 8.46 (d, 2H, *J* = *5.7 Hz*)
*Anal.* C₂₆H₂₀N₂ (360.460) Calc. N 7.77 C 86.64 H 5.59 Found N 7.87 C 86.71 H 5.60

### Preparation of o-bis[1-(6,7-dimethoxyisoquinoly/)]-xylene 2m

Compound 2m was prepared according to the same chemical procedure as described for compound 2a using compound 1a as starting material and using α,α'-dibromo-o-xylene instead of 1,3-diiodopropane. But after alkaline hydrolysis and evaporation of EtOH the residue was dispersed in water (100 mL). The precipitate was filtered, dried and recrystallized from ArMe/n-hexane ; *yield,* 35 % ; *mp* 228-231 °C
*¹H-NMR* (CDCl₃) δ 3.79 (s, 6H), 4.01 (s, 6H), 4.66 (s, 4H), 6.96 (dd, 2H, *J* = *3.4 and 5.6 Hz*), 7.04 (s, 2H), 7.07 (dd, 2H, *J* = *3.4 and 5.6 Hz*), 7.16 (s, 2H), 7.40 (d, 2H, *J* = *5.6 Hz*), 8.30 (d, 2H, *J = 5.6 Hz*)
*Anal*. C₃₀H₂₈N₂O₄ (480.564) Calc. N 5.83 C 74.98 H 5.87 Found N 5.85 C 75.13 H 5.97

### Preparation of m-bis[1-(6,7-dimethoxyisoquinolyl)]-xylene 2n

Compound 2n was prepared according to the same chemical procedure as described for compound 1b using compound 2a as starting material and using α,α'-dibromo-m-xylene instead of 1,3-diiodopropane. But after alkaline hydrolysis and evaporation of EtOH the residue was dispersed in water (100 mL). The precipitate was filtered, dried and recrystallized from ArMe ; *yield,* 31 % ; *mp* 177-179 °C
*¹H-NMR* (CDCl₃) δ 3.62 (s, 6H), 3.99 (s, 6H), 4.49 (s, 4H), 7.01 (s, 2H), 7.09-7.15 (m, 5H), 7.21 (s, 1H), 7.39 (d, 2H, *J* = *5.6 Hz*), 8.33 (d, 2H, *J* = *5.6 Hz*)
*Anal.* C₃₀H₂₈N₂O₄ (480.564) Calc. N 5.83 C 74.98 H 5.87 Found N 5.85 C 75.33 H 5.93

### Preparation of p-bis[1-(6,7-dimethoxyisoquinolyl)]-xylene 2o

Compound 2o was prepared according to the same chemical procedure as described for compound 2a using compound 1b as starting material and using α,α'-dibromo-p-xylene instead of 1,3-diiodopropane. But after alkaline hydrolysis and evaporation of EtOH the residue was dispersed in water (100 mL). The precipitate was filtered, dried and recrystallized from ArMe ; *yield, 37* % ; *mp* 225-227 °C
*¹H-NMR* (CDCl₃) δ 3.79 (s, 6H), 3.99 (s, 6H), 4.52 (s, 4H), 7.02 (s, 2H), 7.16 (s, 4H), 7.23 (s, 2H); 7.39 (d, 2H, *J* = *5.6 Hz*), 8.33 (d, 2H, *J* = *5.6 Hz*)
*Anal.* C₃₀H₂₈N₂O₄ (480.564) Calc. N 5.83 C 74.98 H 5.87 Found N 5.87 C 75.02 H 5.92

### Preparation of o-bis[1-(6,7,8-trimethoxyisoquinolyl)]-xylene 2p

Compound 2p was prepared according to the same chemical procedure as described for compound 2a using compound 1c as starting material and using α,α'-dibromo-o-xylene instead of 1,3-diiodopropane. The crude residue was purified by flash chromatography (MeCOOEt). The resulting solid was recrystallized from MeCOOEt/n-hexane ; *yield*, 24 % ; *mp* 146-148 °C
*¹H-NMR* (CDCl₃) δ 3.57 (s, 6H), 3.87 (s, 6H), 4.00 (s, 6H), 4.91 (s, 4H), 6.58 (dd, 2H, *J* = *3.4 and 5.6 Hz*), 6.90 (s, 2H), 6.92 (dd, 2H, *J* = *3.4 and 5.6 Hz*), 7.41 (d, 2H, *J* = *5.6 Hz*), 8.40 (d, 2H, *J* = 5.6 *Hz*)
*Anal.* C₃₂H₃₂N₂O₆ (540.616) Calc. N 5.18 C 71.10 H 5.97 Found N 5.23 C 71.00 H 6.08

### Preparation of m-bis[1-(6,7,8-trimethoxyisoquinolyl)]-xylene 2q

Compound 2q was prepared according to the same chemical procedure as described for compound 2a using compound 1c as starting material and using α,α'-dibromo-m-xylene instead of 1,3-diiodopropane. The crude residue was purified by flash chromatography (MeCOOEt).The resulting solid was recrystallized from MeCOOEt/n-hexane ; *yield,* 38 % ; *mp* 102-104 °C
*¹H-NMR* (CDCl₃) δ 3.55 (s, 6H), 3.82 (s, 6H), 3.99 (s, 6H), 4.74 (s, 4H), 6.85 (s, 2H), 6.86 (d, 2H, *J* = *7.6 Hz*), 6.93 (s, 1H) 7.05 (t, 1H, *J* = *7.6 Hz*), 7.34 (d, 2H, *J* = *5.6 Hz*), 8.30 (d, 2H, *J* = *5.6 Hz*)
*Anal.* C₃₂H₃₂N₂O₆ (540.616) Calc. N 5.18 C 71.10 H 5.97 Found N 5.19 C 71.01 H 6.20

### Preparation of p-bis[1-(6,7,8-trimethoxyisoquinolyl)]-xylene 2r

Compound 2r was prepared according to the same chemical procedure as described for compound 2a using compound 1c as starting material and using α,α'-dibromo-p-xylene instead of 1,3-diiodopropane. The crude residue was purified by flash chromatography (MeCOOEt). The resulting solid was recrystallized from MeCOOEt/n-hexane ; *yield,* 23 % ; *mp* 176-178 °C
*¹H-NMR* (CDCl₃) δ 3.66 (s, 6H), 3.85 (s, 6H), 3.97 (s, 6H), 4.74 (s, 4H), 6.85 (s, 2H), 6.99 (s, 4H), 7.34 (d, 2H, *J* = *5.6 Hz*), 8.30 (d, 2H, *J = 5.6 Hz*)
*Anal*. C₃₂H₃₂N₂O₆ (540.616) Calc. N 5.18 C 71.10 H 5.97 Found N 5.14 C 71.04 H 6.13

### Preparation of 1,3-bis[1-(8-methylisoquinolyl)]-propane 2s

Compound 2s was prepared according to the same chemical procedure as described for compound 2a using compound 1d as starting material. The resulting solid was recrystallized from ArMe/n-hexane; *yield*, 23 % ;
¹H-NMR (CDCl₃) δ 2.32 (pentuplet, 2H, *J* = *7.8 Hz*), 2.87 (s, 6H), 3.62 (t, 4H, *J* = *7.8 Hz*), 7.33 (d, 2H, *J* = *7.0 Hz*), 7.44-7.47 (m, 4H), 7.62 (d, 2H, *J* = *8.1 Hz*), 8.36 (d, 2H, *J* = *5.5 Hz*)

### Preparation of 1,3-bis[1-(5-bromoisoquinolyl)]-propane 2t

Compound 2t was prepared according to the same chemical procedure as described for compound 2a using compound 1e as starting material. But after alkaline hydrolysis and evaporation of EtOH the residue was dispersed in water (100 mL). The precipitate was filtered, dried and recrystallized from MeCOOEt ; *yield,* 35 % ;
¹H-NMR (CDCl₃) δ 2.32 (pentuplet, 2H, *J* = *7.7 Hz*), 3.62 (t, 4H, *J* = *7.7 Hz*), 7.42 (td, 2H, *J* = *7.6 and 8.3 Hz*), 7.88 (d, 2H, *J* = *5.9 Hz*), 7.94 (dd, 2H, *J* = *0.8 and 7.5 Hz*), 8.18 (d, 2H, *J* = *8.5 Hz*), 8.53 (d, 2H, *J* = *6.1 Hz*)

### Preparation of 1,4-bis[1-(6,7-dimethoyisoquinoly/)]-butene 2u

Compound 2u was prepared according to the same chemical procedure as described for compound 2a using compound 1b as starting material. But the hydrolysis was conducted in EtOH/Me₂CO (1/1) and the alkylated Reissert compound was recrystallized from EtOH before the hydrolysis. The resulting solid was recrystallized from *i*PrOH; *yield,* 30 %
¹H-NMR (CDCl₃) δ 3.97 (s, 6H), 4.03 (s, 6H), 4.24 (d, 4H, *J* = *4.4 Hz*)*,* 5.99 (t, 2H, *J* = *4.4 Hz*)*,* 7.07 (s, 2H), 7.38 (s, 2H), 7.41 (d, 2H, *J* = *5.6 Hz*), 8.32 (d, 2H, *J* = *5.6 Hz*)

### Preparation of 1,1'-bis(1-isoquinolyl)-dimethylether 2v

A solution of 1-hydroxymethyl-isoquinoline 1f (0.62 g ; 3.9 mmol) and 1-chloromethyl-isoquinoline 1g (0.7 g ; 3.9 mmol) in DMF (15 mL) was added dropwise to a stirred suspension of sodium hydride (0.16 g; 3.9 mmol) in DMF (15 mL) at room temperature. The reaction medium was stirred for 2 h and poured into water (200 mL). The aqueous solution was extracted with CHCl₃ (3 x 50 ml). The organic layers were dried over anhydrous MgSO₄ and evaporated under reduced pressure to afford oil. This oil was purified by flash chromatography (Me₂CO) to afford a cream solid (0.8 g); yield 68 %
¹H-NMR (CDCl₃) δ 5.40 (s, 4H), 7.58 (t, 2H, *J* = *7.6 Hz*), 7.68 (d, 2H, *J* = *5.8 Hz),* 7.72 (t, 2H, *J* = *7.6 Hz*), 7.85 (d, 2H, *J* = *8.2 Hz*), 8.33 (d, 2H, *J* = *8.4 Hz*), 8.50 (d, 2H, *J* = *5.8 Hz*)

### Preparation of 1,3-bis[1-(2-methylisoguinolydium)]-propane diiodide 3a

A solution of compound 2a (1.3 g ; 4.4 mmol) in DMF (10 mL) was heated until dissolution with an excess of methyl iodide (1.0 mL ; 16 mmol). After 2 h, Et₂O was added resulting of a rapid crystallization of yellow solid. The precipitate was filtered off, washed with Et₂O (2 x 10 mL) and dried (2.3g) ; *yield,* 89 % ; *mp* 237-238 °C dec.
*¹H-NMR* (DMSO) δ 2.11 (br m, 2H), 4.08 (t, 4H, *J* = *8.3 Hz*), 4.54 (s, 6H), 8.12 (t, 2H, *J* = *7.7 Hz*), 8.23 (t, 2H, *J* = *7.7 Hz*)*,* 8.31 (d, 2H, *J* = *8.1 Hz*), 8.43 (d, 2H, *J = 6.9 Hz*), 8.71 (d, 2H, *J* = *6.9 Hz*)*,* 9.00 (d, 2H, *J* = *8.6 Hz*)
*Anal.* C₂₃H₂₄N₂I₂.¼H₂O (586.769) Calc. N 4.77 C 47.08 H 4.21 Found N 4.87 C 46.78 H 3.83

### Preparation of 1,4-bis[1-(2-melhylisoquinolylium)]-butane diiodide 3b

Compound 3b was prepared according to the same chemical procedure as described for compound 3a using compound 2b as starting material ; *yield,* 98 % ; *mp* 297-298 °C dec.
*¹H-NMR* (DMSO) δ 2.06 (br s, 4H), 3.72 (br s, 4H), 4.51 (s, 6H), 8.07 (t, 2H, *J* = *7.5 Hz*), 8.23 (t, 2H, *J* = *7.5 Hz*), 8.32 (d, 2H, *J* = *8.1 Hz*), 8.44 (d, 2H, *J* = *6.9 Hz*), 8.71 (d, 2H, *J* = *6.9 Hz*), 8.82 (d, 2H, *J* = *8.6 Hz*)
*Anal.* C₂₄H₂₆N₂I₂ (596.286) Calc. N 4.70 C 48.34 H 4.40 Found N 4.85 C 48.24 H 4.37

### Preparation of 1,5-bis[1-(2 -methylisoquinolylium)]-pentane diiodide 3c

Compound 3c was prepared according to the same chemical procedure as described for compound 3a using compound 2c as starting material ; *yield,* 73 % : *mp* 245-247 °C dec.
*¹H-NMR* (DMSO) δ 1.85 (br m, 6H), 3.65 (br t, 4H), 4.46 (s, 6H), 8.04 (t, 2H, *J* = *7.6 Hz*), 8.21 (t, 2H, *J* = *7.6 Hz*), 8.30 (d, 2H, *J* = *8.2 Hz*)*,* 8.41 (d, 2H, *J* = *6.9 Hz*)*,* 8.69 (d, 2H, *J* = *6.9 Hz*), 8.72 (d, 2H, *J* = 8.7 *Hz*)
*Anal.* C₂₅H₂₈N₂I₂ (610.313) Calc. N 4.59 C 49.20 H 4.62 Found N 4.61 C 48.98 H 4.73

### Preparation of 1,3-bis[1-(6,7-dimethoxy-2-methylisoquinolylium)]-propane diiodide 3d

Compound 3d was prepared according to the same chemical procedure as described for compound 3a using compound 2d as starting material ; *yield,* 97 % ; *mp* 249-251 °C dec.
*¹H-NMR* (DMSO) δ 2.09 (br m, 2H), 4.00 (t, 4H, *J* = *8.2 Hz*), 4.03 (s, 6H), 4.07 (s, 6H), 4.44 (s, 6H), 7.69 (s, 2H), 7.84 (s, 2H), 8.12 (d, 2H, *J* = *6.8 Hz*), 8.49 (d, 2H, *J* = *6.8 Hz*)
*Anal.* C₂₇H₃₂N₂O₄I₂h.H₂O (720.378) Calc. N 3.89 C 45.02 H 4.76 Found N 4.05 C 45.11 H 4.48

### Preparation of 1,4-bis[[1-(6,7-dimethoxy-2-methylisoquinolylium)]-butane diiodide 3e

Compound 3e was prepared according to the same chemical procedure as described for compound 3a using compound 2e as starting material ; *yield,* 99 % ; *mp* 290-291 °C dec.
*¹H-NMR* (DMSO) δ 2.01 (br s, 4H), 3.65 (br s, 4H), 4.04 (s, 6H), 4.05 (s, 6H), 4.40 (s, 6H), 7.72 (s, 2H), 7.73 (s, 2H), 8.14 (d, 2H, *J* = *6.8 Hz*), 8.49 (d, 2H, *J* = *6.8 Hz*)
*Anal.* C₂₈H₃₄N₂O₄I₂.H₂O (734.405) Calc. N 3.81 C 45.79 H 4.94 Found N 4.13 C 45.39 H 4.60

### Preparation of 1,5-bis[1-(6,7-dimethoxy-2-methylisoquinolylium)]-pentane diiodide 3f

Compound 3f was prepared according to the same chemical procedure as described for compound 3a using compound 2f as starting material ; *yield,* 93 % ; *mp* 256-257 °C dec.
*¹H-NMR* (DMSO) δ 1.72 (br m, 2H), 1.81 (br m, 4H), 3.57 (t, 4H, *J* = *7.6 Hz*), 3.95 (s, 6H), 4.05 (s, 6H), 4.36 (s, 6H), 7.63 (s, 2H), 7.70 (s, 2H), 8.13 (d, 2H, *J* = *6.8 Hz*), 8.48 (d, 2H, *J* = *6.8 Hz*)
*Anal.* C₂₉H₃₆N₂O₄I₂.1½H₂O (757.440) Calc. N 3.70 C 45.99 H 5.19 Found N 3.91 C 46.10 H 4.80

### Preparation of 1,3-bis[1-(6,7,8-trimethoxy-2-methylisoquinolylium)]-propane diiodide 3g

Compound 3g was prepared according to the same chemical procedure as described for compound 3a using compound 2g as starting material ; *yield*, 75 % ; *mp* 237-238 °C dec.
*¹H-NMR* (DMSO) δ 2.04 (br m, 2H), 3.84 (br s, 4H), 3.90 (s, 6H), 4.06 (s, 6H), 4.08 (s, 6H), 4.43 (s, 6H), 7.59 (s, 2H), 8.14 (d, 2H, *J* = *6.9 Hz*), 8.51 (d, 2H, *J* = *6.9 Hz*)
*Anal.* C₂₉H₃₆N₂O₆I₂ (762.415) Calc. N 3.67 C 45.69 H 4.76 Found N 3.79 C 45.41 H 4.43

### Preparation of 1,4-bis[1-(6,7,8-trimethoxy-2-methylisoquinolylium)]-butane diiodide 3h

Compound 3h was prepared according to the same chemical procedure as described for compound 3a using compound 2h as starting material ; *yield,* 95 % ; *mp* 150-152 °C dec.
*¹H-NMR* (DMSO) δ 1.95 (br s, 4H), 3.64 (br s, 4H), 3.92 (s, 6H), 4.08 (s, 6H), 4.10 (s, 6H), 4.40 (s, 6H), 7.61 (s, 2H), 8.15 (d, 2H, *J* = *6.9 Hz*), 8.51 (d, 2H, *J* = *6.9 Hz*)
*Anal*. C₃₀H₃₈N₂O₆I₂ (776.442) Calc. N 3.61 C 46.41 H 4.93 Found N 3.82 C 45.65 H 4.68

### Preparation of o-bis[1-(2-methylisoquinolylium)]-xylene diiodide 3j

Compound 3j was prepared according to the same chemical procedure as described for compound 3a using compound 2j as starting material ; *yield,* 84 % ; *mp* 292-293 °C dec.
*¹H-NMR* (DMSO) δ 4.47 (s, 6H), 5.46 (s, 4H), 6.25 (dd, 2H, *J* = 3.4 *and 5.5 Hz*), 7.03 (dd, 2H, *J* = *3.4 and 5.5 Hz*), 8.09 (t, 2H, *J* = *7.6 Hz*), 8.29 (t, 2H, *J* = *7.6 Hz*), 8.44 (d, 2H, *J* = *8.2 Hz*), 8.65 (d, 2H, *J* = 6.8 *Hz*), 8.78 (d, 2H, *J* = *8.6 Hz*), 8.90 (d, 2H, *J* = 6.8 *Hz*)
*Anal.* C₂₈H₂₆N₂I₂ (644.330) Calc. N 4.35 C 52.19 H 4.07 Found N 4.52 C 52.31 H 3.82

### Preparation of m-bis[1-(2-methylisoquinolylium)]-xylene diiodide 3k

Compound 3k was prepared according to the same chemical procedure as described for compound 3a using compound 2k as starting material ; *yield,* 86 % ; *mp* 244-247 °C dec.
*¹H-NMR* (DMSO) δ 4.32 (s, 6H), 5.07 (s, 4H), 6.76 (s, 1H), 7.05 (d, 2H, *J* = *8.1 Hz),* 7.29 (t, 1H, *J* = *8.1 Hz*), 7.89 (t, 2H, *J* = *7.7 Hz*), 8.20 (t, 2H, *J* = *7.7 Hz*), 8.33 (d, 2H, *J* = *8.0 Hz*), 8.49 (d, 2H, *J* = *6.3 Hz*), 8.56 (d, 2H, *J* = *8.6 Hz*), 8.72 (d, 2H, *J* = *7.4 Hz*)
*Anal.* C₂₈H₂₆N₂I₂.½H₂O (653.338) Calc. N 4.29 C 51.43 H 4.13 Found N 4.35 C 51.54 H 3.69

### Preparation of p-bis[1-(2-methylisoquinolylium)]-xylene diiodide 31

Compound 31 was prepared according to the same chemical procedure as described for compound 3a using compound 21 as starting material ; *yield,* 84 % ; *mp* > 300 °C
*¹H-NMR* (DMSO) δ 4.35 (s, 6H), 5.12 (s, 4H), 7.08 (s, 4H), 8.00 (t, 2H, *J* = *7.7 Hz*), 8.21 (t, 2H, *J* = *7.7 Hz*), 8.33 (d, 2H, *J* = *8.2 Hz*), 8.51 (d, 2H, *J = 6.9 Hz*), 8.68 (d, 2H, *J* = *8.7 Hz*), 8.73 (d, 2H, *J* = *6.9 Hz*)
*Anal.* C₂₈H₂₆N₂I₂ (644.330) Calc. N 4.35 C 52.19 H 4.07 Found N 4.69 C 52.17 H 3.85

### Preparation of o-bis[1-(6,7-dimethoxy-2-methylisoquinolylium)]-xylene diiodide 3m

Compound 3m was prepared according to the same chemical procedure as described for compound 3a using compound 2m as starting material ; *yield,* 99 % ; *mp* 277-279 °C
*¹H-NMR* (DMSO) δ 3.92 (s, 6H), 4.09 (s, 6H), 4.35 (s, 6H), 5.41 (s, 4H), 6.24 (dd, 2H, *J* = *3.5 and 5.6 Hz*), 7.05 (dd, 2H, *J* = *3.5 and 5.6 Hz*), 7.71 (s, 2H), 7.85 (s, 2H), 8.35 (d, 2H, *J* = *6.8 Hz*), 8.68 (d, 2H, *J* = *6.8 Hz*)
*Anal.* C₃₂H₃₄N₂O₄I₂ (764.434) Calc. N 3.66 C 50.28 H 4.28 Found N 3.84 C 50.15 H 4.34

### Preparation of m-bis[1-(6,7-dimethoxy-2-methylisoquinolylium)]-xylene diiodide 3n

Compound 3n was prepared according to the same chemical procedure as described for compound 3a using compound 2n as starting material ; *yield,* 99 % ; *mp* 266-267 °C dec.
*¹H-NMR* (DMSO) δ 3.54 (s, 6H), 4.11 (s, 6H), 4.17 (s, 6H), 4.90 (s, 4H), 6.31 (s, 1H), 7.40 (s, 4H), 7.45 (t, 1H, *J* = *7.9 Hz*), 7.68 (s, 2H), 8.17 (d, 2H, *J* = *6.8 Hz*), 8.45 (d, 2H, *J* = *6.8 Hz*)
*Anal.* C₃₂H₃₄N₂O₄I₂.½H₂O (773.442) Calc. N 3.62 C 49.69 H 4.56 Found N 4.12 C 49.69 H 4.33

### Preparation of p-bis[1-(6,7-dimethoxy-2-methylisoquinolylium)]-xylene diiodide 3o

Compound 3o was prepared according to the same chemical procedure as described for compound 3a using compound 2o as starting material ; *yield,* 97 % ; *mp* 291-292 °C
*¹H-NMR* (DMSO) δ 3.88 (s, 6H), 4.05 (s, 6H), 4.26 (s, 6H), 5.05 (s, 4H), 7.12 (s, 4H), 7.74 (s, 2H), 7.77 (s, 4H), 8.21 (d, 2H, *J* = *6.8 Hz*), 8.52 (d, 2H, *J* = *6.8 Hz*) *Anal.* C₃₂H₃₄N₂O₄I₂ (764.434) Calc. N 3.66 C 50.28 H 4.28 Found N 3.97 C 49.99 H 4.30

### Preparation of o-bis[1-(6,7,8-trimethoxy-2-methylisoquinolylium)]-xylene diiodide 3p

Compound 3p was prepared according to the same chemical procedure as described for compound 3a using compound 2p as starting material ; *yield* 70 % *mp* 197-198 °C dec.
*¹H-NMR* (DMSO) δ 3.63 (s, 6H), 3.91 (s, 6H), 4.12 (s, 6H), 4.33 (s, 6H), 5.21 (br s, 4H), 6.58 (dd, 2H, *J* = *3.5 and 5.8 Hz*), 7.14 (dd, 2H, *J* = *3.5 and 5.8 Hz*), 7.71 (s, 2H), 8.34 (d, 2H, *J* = *6.9 Hz*), 8.69 (d, 2H, *J* = *6.9 Hz*)
*Anal.* C₃₄H₃₈N₂O₆I₂.½H₂O (833.494) Calc. N 3.36 C 49.00 H 4.72 Found N 3.48 C 49.03 H 4.60

### Preparation of m-bis[1-(6,7,8-trimethoxy-2-methylisoquinolylium)]-xylene diiodide 3q

Compound 3q was prepared according to the same chemical procedure as described for compound 3a using compound 2q as starting material ; *yield,* 98 % ; *mp* 202-204 °C dec.
*¹H-NMR* (DMSO) δ 3.60 (s, 6H), 3.83 (s, 6H), 4.11 (s, 6H), 4.17 (s, 6H), 5.07 (br s, 4H), 6.65 (s, 1H), 6.97 (d, 2H, *J* = *7.7 Hz*), 7.29 (t, 1H, *J* = *7.7 Hz*), 7.62 (s, 2H), 8.21 (d, 2H, *J* = *6.9 Hz*), 8.53 (d, 2H, *J* = *6.9 Hz*)
*Anal.* C₃₄H₃₈N₂I₆I₂ (824.486) Calc. N 3.40 C 49.53 H 4.65 Found N 3.67 C 49.68 H 4.44

### Preparation of p-bis[1-(6,7,8-trimethoxy-2-methylisoquinolylium)]-xylene diiodide 3r

Compound 3r was prepared according to the same chemical procedure as described for compound 3a using compound 2r as starting material ; *yield,* 98 % ; *mp* 226-227 °C dec.
*¹H-NMR* (DMSO) δ 3.66 (s, 6H), 3.85 (s, 6H), 4.08 (s, 6H), 4.19 (s, 6H), 5.10 (br s, 4H), 7.04 (s, 4H), 7.63 (s, 2H), 8.23 (d, 2H, *J* = *6.9 Hz),* 8.55 (d, 2H, *J* = *6.9 Hz*)
*Anal.* C₃₄H₃₈N₂O₆I₂ (824.486) Calc. N 3.40 C 49.53 H 4.65 Found N 3.69 C 49.38 H 4.29

### Preparation of 1,3-bis[1-(8-methylisoquinolylium)]-propane diiodide 3s

Compound 3s was prepared according to the same chemical procedure as described for compound 3a using compound 2s as starting material; *yield,* 92 % ¹H-NMR (DMSO-d₆) δ 2.13 (br pentuplet, 2H), 3.04 (s, 6H), 4.02 (t, 4H, *J* = *8.3 Hz*), 4.53 (s, 6H), 7.89 (d, 2H, *J* = *7.1 Hz*), 8.03 (t, 2H, *J* = *7.5 Hz*), 8.12 (d, 2H, *J* = *7.8 Hz*), 8.39 (d, 2H, *J* = *6.8 Hz*), 8.70 (d, 2H, *J* = *6.8 Hz*)

### Preparation of 1,3-bis[1-(5-bromoisoquinolylium)]-propane diiodide 3t

Compound 3t was prepared according to the same chemical procedure as described for compound 3a using compound 2t as starting material ; *yield,* 88 % ¹H-NMR (DMSO-d₆) δ 2.09 (br pentuplet, 2H), 4.07 (t, 4H, *J* = *8.4 Hz*), 4.57 (s, 6H), 8.01 (t, 2H, *J* = *8.1 Hz*), 8.05 (d, 2H, *J* = *7.1 Hz*), 8.58 (d, 2H, *J* = *7.5 Hz*), 8.80 (d, 2H, *J* = *7.2 Hz*), 9.03 (d, 2H, *J* = *8.7 Hz*)

### Preparation of 1,1'-bis[1-(2-methylisoquinolylium)]-dimethylether 3v

A solution of compound 2v (0.9 g ; 3.0 mmol) with an excess of methyl iodide (1.0 mL ; 16 mmol) in DMSO (10 mL) was heated until dissolution. After 2 h, Et₂O was added resulting of a dark red solid. The solid was triturated with MeOH and filtered off, washed with Et₂O (2 x 10 mL) and dried (1.5 g) to afford a yellow solid; *yield,* 86 %

¹H-NMR (DMSO) δ 4.55 (s, 6H), 5.84 (s, 4H), 8.12 (t, 2H, *J* = *8.4 Hz*), 8.25 (t, 2H, *J* = *7.5 Hz*), 8.34 (d, 2H, *J* = *8.2 Hz*), 8.60 (d, 2H, *J* = *6.8 Hz*), 8.79 (d, 2H, *J* = *6.8 Hz*), 9.00 (d, 2H, *J* = *8.5 Hz*)

### Preparation of 1,3-bis[1-(2-methyl-1,2,3,4-tetrahydrolisoquinolyl)]-propane hydrochloride 4a

Under inert atmosphere, NaBH₄ (0.97 g ; 25.5 mmol) was added to a solution of compound 3a (1.0 g ; 1.7 mmol) in MeOH (100 mL) at room temperature. After 15 min, MeOH were removed under reduced pressure and the crude residue was dissolved in a 1N aqueous HCl (100 mL). The acidic layer was washed with Et₂O (3 x 20 ml) and then basified with NH₄OH. The suspension was extracted with CH₂Cl₂ (3 x 30 mL). The organic layers were collected, dried over anhydrous MgSO₄ and evaporated under reduced pressure to afford a colourless oil which was purified by flash chromatography (Me₂CO/MeOH, 9/1). The resulting oil was isolated as hydrochloride salt and recrystallized from EtOH/Et₂O (0.55 g) ; *yield*, 78 %
*Anal.* C₃₃H₃₂N₂Cl₂.¼H₂O (411.927) Calc. N 6.80 C 67.06 H 7.95 Found N 6.83 C 66.78 H 8.31

### Preparation of 1,4-bis[1-(2-methyl-1,2,3,4-tetrahydroisoquinolyl)]-butane hydrochloride 4b

Compound 4b was prepared according to the same chemical procedure as described for compound 4a (without flash chromatography) using compound 3b as starting material. The crude salt was recrystallized from EtOH/Et₂O ; *yield*, 82 %
*Anal.* C₂₄H₃₄N₂Cl₂.¾H₂O (434.962) Calc. N 6.44 C 66.27 H 8.23 Found N 6.57 C 67.40 H 8.56

### Preparation of 1,5-bis[1-(2-methyl-1,2,3,4-tetrahydroisoquinolyl)]-pentane hydrochloride 4c

Compound 4c was prepared according to the same chemical procedure as described for compound 4a (without flash chromatography) using compound 3c as starting material. The crude salt was recrystallized from EtOH/Et₂O ; *yield,* 81 %
*Anal.* C₂₅H₃₆N₂Cl₂.H₂O (453.493) Calc. N 6.18 C 66.21 H 8.45 Found N 6.20 C 66.19 H 8.79

### Preparation of 1,3-bis[1-(6,7-dimethoxy-2-methyl-1,2,3,4-tetrahydroisoquinolyl)]-propane hydrochloride 4d

Compound 4d was prepared according to the same chemical procedure as described for compound 4a (without flash chromatography) using compound 3d as starting material. The crude salt was recrystallized from MeCN/Et₂O ; *yield,* 67 %
*Anal.* C₂₇H₄₀N₂O₄Cl₂.¾H₂O (541.039) Calc. N 5.18 C 59.94 H 7.73 Found N 5.18 C 60.17 H
7.68

The stereoisomers were separated by semi-preparative HPLC with n-hexane/isopropanol (8/2) + 0.05 % DEA
Retention time : E1: 7.3 min ; Meso: 10.6 min ; E2: 21.3 min
Purity (HPLC) E1: 97.4 %, Meso: 98.7 %, E2: 95.9 %
Stereoisomeric excess (HPLC) E1: 99.7 %, Meso: 99.6 %, E2: 99.6 %

### Preparation of 1,4-bis[1-(6,7-dimethoxy-2-methyl-1,2,3,4-tetrahydroisoquinolyl)]-butane hydrochloride 4e

Compound 4e was prepared according to the same chemical procedure as described for compound 4a (without flash chromatography) using compound 3e as starting material. The crude salt was recrystallized from EtOH/Et₂O ; *yield,* 84 *% ; Anal.* C₂₈H₄₂N₂O₄Cl₂.H₂O (559.570) Calc. N 5.01 C 60.10 H 7.93 Found N 4.90 C 60.06 H 7.76
The stereoisomers were separated by semi-preparative HPLC with n-hexane/isopropanol (9/1) + 0.05 % DEA
Retention time : E1: 9.9 min ; Meso: 18.1 min ; E2: 32.8 min
¹H-NMR (CDCl₃)
Purity (HPLC) E1: 98.4 %, Meso: 99.8 %, E2: 96.5 %
Stereoisomeric excess (HPLC) E1: 99.7 %, Meso: 100.0 %, E2: 99.9 %

### Preparation of 1,5-bis[1-(6,7-dimethoxy-2-methyl-1,2,3,4-tetrahydroisoquinolyl)]-pentane hydrochloride 4f

Compound 4f was prepared according to the same chemical procedure as described for compound 4a (without flash chromatography) using compound 3f as starting material. The crude salt was recrystallized from EtOH/Et₂O ; *yield*, 83 %
*Anal.* C₂₉H₄₄N₂O₄Cl₂.H₂O (573.597) Calc. N 4.88 C 60.72 H 8.08 Found N 4.75 C 60.47 H 8.31

The stereoisomers were separated by semi-preparative HPLC with n-hexane/isopropanol (8/2) + 0.05 % DEA
Retention time : E1: 6.3 min ; Meso: 10.5 min ; E2: 20.1 min
Purity (HPLC) E1: 93.7 %, Meso: 99.3 %, E2: 97.0 %
Stereoisomeric excess (HPLC) E1: 99.9 %, Meso: 99.5 %, E2: 98.6 %

### Preparation of 1,3-bis[1-(6,7,8-trimethoxy-2-methyl-1,2,3,4-tetrahydroisoquinolyl)]-propane hydrochloride 4g

Compound 4g was prepared according to the same chemical procedure as described for compound 4a (without flash chromatography) using compound 3g as starting material. The crude salt was recrystallized from 1,4-dioxane/Et₂O ; *yield, 70* % ; *Anal.* C₂₉H₄₄N₂O₆Cl₂.1½H₂O (614.603) Calc. N 4.56 C 56.67 H 7.71 Found N 4.58 C 56.37 H 8.02

The stereoisomers were separated by semi-preparative HPLC with n-hexane/isopropanol (9/1) + 0.05 % DEA
Retention time : E1: 6.1 min ; Meso: 10.2 min ; E2: 13.1 min
Purity (HPLC) E1: 99.7 %, Meso: 99.0 %, E2: 97.7 %
Stereoisomeric excess (HPLC) E1: 100.0 %, Meso: 99.7 %, E2: 99.8 %

### Preparation of 1,4-bis[1-(6,7,8-trimethoxy-2-methyl-1,2,3,4-tetrahydroisoquinolyl)]-butane hydrochloride 4h

Compound 4h was prepared according to the same chemical procedure as described for compound 4a (without flash chromatography) using compound 3h as starting material. The crude salt was recrystallized from EtOH/Et₂O ; *yield,* 84 % ; *Anal.* C₃₀H₄₆N₂O₆Cl₂.½H₂O (610.613) Calc. N 4.59 C 59.01 H 7.76 Found N 4.68 C 59.16 H 7.98

The stereoisomers were separated by semi-preparative HPLC with n-hexane/isopropanol (9/1) + 0.05 % DEA
Retention time: E1: 5.6 min ; Meso: 9.7 min ; E2: 13.3 min
Purity (HPLC) E1: 99.4 %, Meso: 98.6 %, E2: 97.2 %
Stereoisomeric excess (HPLC) E1: 100.0 %, Meso: 99.8 %, E2: 98.5 %

### Preparation of o-bis[1-(2-methyl-1,2,3,4-tetrahydroquinolyl)]-xylene hydrochloride 4j

Compound 4j was prepared according to the same chemical procedure as described for compound 4a (flash chromatography with Me₂CO) using compound 3j as starting material. The hydrochloride salt was recrystallized from 1,4-dioxane/Et₂O ; *yield,* 33 % *Anal.* C₂₈H₃₄N₂Cl₂.1½H₂O (496.518) Calc. N 5.64 C 67.73 H 7.51 Found N 5.72 C 68.13 H 7.56

### Preparation of m-Bis[1-(2-methyl-1,2,3,4-tetrahydroisoquinolyl)]-xylene hydrochloride 4k

Compound 4k was prepared according to the same chemical procedure as described for compound 4a (flash chromatography with Me₂CO) using compound 3k as starting material. The hydrochloride salt was recrystallized from EtOH/Et₂O ; *yield*, 44 %
*Anal.* C₂₈H₃₄N₂Cl₂ (469.494) Calc. N 5.97 C 71.63 H 7.30 Found N 5.96 C 71.36 H 7.65

### Preparation of p-bis[1-(2-methyl-1,2,3,4-tetrahydroisoquinolyl)]-xylene hydrochloride 4l

Compound 41 was prepared according to the same chemical procedure as described for compound 4a (flash chromatography with Me₂CO) using compound 31 as starting material. The hydrochloride salt was recrystallized from EtOH/Et₂O ; *yield*, 40 %
*Anal.* C₂₈H₃₄N₂Cl₂.2H₂O (505.526) Calc. N 5.54 C 66.53 H 7.57 Found N 5.53 C 66.53 H 7.25

### Preparation of o-bis[1-(6,7-dimethoxy-2-methyl-1,2,3,4-tetrahyroisoquinolyl)]-xylene hydrochloride 4m

Compound 4m was prepared according to the same chemical procedure as described for compound 4a (flash chromatography with Me₂CO) using compound 3m as starting material. The salt was recrystallized from 1,4-dioxane/Et₂O ; yield, 30 %
*Anal.* C₃₂H₄₂N₂O₄Cl₂.2H₂O (625.630) Calc. N 4.48 C 61.43 H 7.41 Found N 4.59 C 61.67 H 7.44

### Preparation of m-bis[1-(6,7-dimethoxy-2-methyl-1,2,3,4-tetrahydroisoquinolyl)]-xylene hydrochloride 4n

Compound 4n was prepared according to the same chemical procedure as described for compound 4a (flash chromatography with Me₂CO) using compound 3n as starting material. The salt was recrystallized from dioxane/Et₂O ; yield, 80 %
*Anal.* C₃₂H₄₂N₂O₄Cl₂.2H₂O (625.630) Calc. N 4.48 C 61.43 H 7.41 Found N 4.53 C 61.52 H 7.47

The stereoisomers were separated by semi-preparative HPLC with MeCN + 0.05 % DEA
Retention time : E1: 5.1 min ; Meso: 8.4 min ; E2: 11.8 min
Purity (HPLC) E1: 98.7 %, Meso: 96.6 %, E2: 95.5 %
Stereoisomeric excess (HPLC) E1: 99.6 %, Meso: 99.6 %, E2: 99.2 %

### Preparation of p-bis[1-(6,7-dimethox-2-2-methyl-1,2,3,4-tetrahydroisoquinolyl)]-xylene 4o

Compound 4o was prepared according to the same chemical procedure as described for compound 4a (flash chromatography with Me₂CO) using compound 3o as starting material. The free base was recrystallized from petroleum ether 100-140 °C ; *yield,* 92 % *Anal.* C₃₂H₄₀N₂O₄ (516.682) Calc. N 5.42 C 74.39 H 7.80 Found N 5.43 C 74.75 H 8.16

The stereoisomers were separated by semi-preparative HPLC with n-hexane/isopropanol (7/3) + 0.05 % DEA
Retention time : E1: 8.7 min ; Meso: 17.7 min ; E2: 30.6 min
Purity (HPLC) E1: 99.5 %, Meso: 98.9 %, E2: 96.6 %
Stereoisomeric excess (HPLC) E1: 99.9 %, Meso: 99.9 %, E2: 98.6 %

### Preparation of o-bis[1-(6,7,8-trimethoxy-2-methyl-1,2,3,4-tetrahydroisoquinolyl)]-xylene hydrochloride 4p

Compound 4p was prepared according to the same chemical procedure as described for compound 4a (without flash chromatography) using compound 3p as starting material. The crude hydrochloride salt was recrystallized from 1,4-dioxane/Et₂O ; *yield*, 89 %
*Anal.* C₃₄H₄₆N₂O₆Cl₂.1½H₂O (676.674) Calc. N 4.14 C 60.35 H 7.30 Found N 4.23 C 60.19 H 7.37

### Preparation of m-bis[1-(6,7,8-trimethoxy-2-methyl-1,2,3,4-tetrahydroisoquinolyl)]-xylene hydrochloride 4q

Compound 4q was prepared according to the same chemical procedure as described for compound 4a (without flash chromatography) using compound 3q as starting material. The crude hydrochloride salt was recrystallized from MeCOEt/Et₂O ; *yield,* 98 % *Anal.* C₃₄H₄₆N₂O₆Cl₂.H₂O (667.666) Calc. N 4.19 C 61.16 H 7.24 Found N 4.15 C 61.33 H 7.21

### Preparation of p-bis[1-(6,7,8-trimethoxy-2-methyl-1,2,3,4-tetrahydroisoquinolyl)]-xylene 4r

Compound 4r was prepared according to the same chemical procedure as described for compound 4a (flash chromatography with Me₂CO) using compound 3r as starting material. After chromatography the free base was triturated with Et₂O resulting in rapid crystallization ; *yield,* 64 % *Anal.* C₃₄H₄₆N₂O₆ (576.734) Calc. N 4.86 C 70.81 H 7.69 Found N 4.92 C 70.60 H 8.06

### Preparation of 1,3-bis[1-(2,8-dimethyl-1,2,3,4-tetrahydroisoquinolyl)]-propane hydrochloride 4s

Compound 4s was prepared according to the same chemical procedure as described for compound 4a (without flash chromatography) using compound 3s as starting material. The crude salt was recrystallized from EtOH/Et₂O ; *yield,* 52 %
¹H-NMR (CDCl₃) δ 1.57-1.81(br m, 6H), 2.24 (s, 6H), 2.48 (br s, 6H), 2.60-2.62 (br m, 2H), 2.83 (br t, 2H), 2.93-3.00 (m, 2H), 3.37 (br s, 2H), 3.67 (br s, 2H), 3.92 (d, 2H, *J* = *7.4 Hz*), 6.98 (d, 2H, *J* = *7.4 Hz*), 7.05 (t, 2H, *J* = *7.4 Hz*)

### Preparation of 1,3-bis[1-(5-bromo-2-methyl-1,2,3,4-tetrahyroisoquinolyl)]-propane hydrochloride 4t

Compound 4t was prepared according to the same chemical procedure as described for compound 4a (without flash chromatography) using compound 3t as starting material. The crude salt was recrystallized from EtOH/E₁O; *yield*, 58 %
¹H-NMR (CDCl₃) δ 1.45-1.52 (m, 2H), 1.61-1.70 (m, 2H), 1.77-1.82 (m, 2H), 2.42 (s, 6H), 2.65 (dd, 2H, *J* = *6.8 and 14.9 Hz*), 2.75-2.84 (m, 4H), 3.14-3.46 (m, 2H), 3.46 (br d, 2H), 6.99-7.03 (m, 4H), 7.38-7.41 (m, 2H)

### Preparation of 1,1'-bis[1-(2-methyl-1,2,3,4-tetrnhydroisoquinolyl)]-dimethylether 4v

Compound 4v was prepared according to the same chemical procedure as described for compound 4a using compound 3v as starting material; *yield,* 64 %
¹H-NMR (CDCl₃) δ 2.51 (s, 3H, diastereo A), 2.54 (s, 3H, diastereo B), 2.66-2.72 (m, 2H), 2.75-2.83 (m, 4H), 3.09-3.14 (m, 2H), 3.57-3.61 (m, 2H), 3.70-3.77 (m, 4H), 7.07-7.14 (m, 8H)

### Pharmacological Results

### Radioligand binding studies and data analysis

These experiments are used to evaluate the potency of the bis-tetrahydroisoquinoline derivatives to interact with the apamin-sensitive sites of the SK channels, the selected target for which a blockade is expected to be useful in the treatment of Alzheimer's disease, Parkinson's disease, schizophrenia, cognitive dysfunction, or depression. Two reference compounds are also tested for comparison

### 1. Synaptosome preparation

Synaptosomes are molecules aggregates comprising different proteins, particularly the ones belonging to the SK channels.
Rats (male Wistar, ± 250 g) were killed by decapitation and the brains were quickly removed and kept on ice during dissection. Crude cortex was dispersed in 0.32 M sucrose by using a Potter^{®} homogenizer. After a first centrifugation at 1500 x g for 10 min, the supernatant was centrifuged at 25000 x g for 10 min. The resulting pellet was dispersed in 5 mL 0.32 M sucrose to be aliquoted. Protein concentration was determined by the method of Hartree with bovine serum albumin as a standard.³⁶

### 2. Binding experiments

The buffer consisted of a 10 mM Tris-HCl (pH 7.5) solution containing 5.4 mM KCl and 0.1 % bovine serum albumin. The radioligand was ¹²⁵I-apamin (Perkin-Elmer, Specific activity 81.4 TBq mmol⁻¹). Glass fibre filters (Whatman GF/C) used in these experiments were coated for 1h in 0.5 % polyethylenimine and then washed with 2.5 mL of the ice-cold buffer just before use. Binding experiments were always terminated as follows. Aliquots were filtered under reduced pressure through Whatman filters. Filters were rapidly washed twice with 2.5 mL of buffer. The radioactivity remaining on the filter was evaluated with a Packard Tri-Carb 1600TR liquid scintillation analyser with an efficacy of 69 %. ¹²⁵I-apamin binding to the filters was also estimated in the absence of synaptosomes. This binding was also substracted from the total binding. Curve fitting was carried out using GraphPad Prism^{®}.
*Saturation Binding Experiment -* Synaptosomes (0.2 mg of protein/mL) were incubated with increasing concentrations of ¹²⁵I-apamin (25 µL) with 975 µL of incubation buffer for 1 h at 0°C. Samples were then filtered on Whatman GF/C filter and the radioactivity was measured as described above. Non specific binding was determined in parallel experiments in the presence of an excess of unlabeled apamin (0.1 µM) and substracted from the total binding to obtain the specific binding.
*Competition Experiments between ¹²⁵I-apomin and Bis-tetrahydroisoquinoline derivatives -* Synaptosomes (0.2 mg of protein/mL) were incubated for 1 h at 0°C with ± 10 pM of ¹²⁵I-apamin (25 µL) and nine concentrations of bis-tetrahydroisoquinoline derivatives (10⁻⁴ to 10⁻⁷ M). Non specific binding was determine in the presence of an excess of unlabeled apamin (0.1 µM). Samples were then filtered on Whatman filter and the radioactivity was measured as described above.

**Table 1: Screening of Compounds 4a-r for Affinity to Rat Cortical Apamin Sensitive Sites in Comparison with N-Methyl-Laudanosine (NML)**

| | | | | |
|---|---|---|---|---|
| 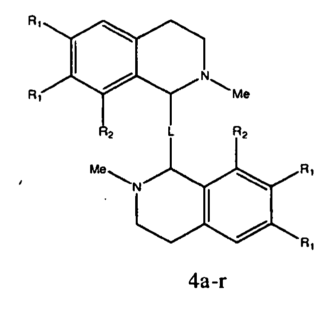 | | | | |

| *N*° | *R₁* | *R₂* | *L* | *%^{a}* |
|---|---|---|---|---|
| *NML* | - | - | - | 54 |
| *4a* | H | H | (CH₂)₃ | 13 |
| *4b* | H | H | (CH₂)₄ | 16 |
| *4c* | H | H | (CH₂)₅ | 9 |
| *4d* | OMe | H | (CH₂)₃ | 82 |
| *4e* | OMe | H | (CH₂)₄ | 72 |
| *4f* | OMe | H | (CH₂)₅ | 78 |
| *4g* | OMe | OMe | (CH₂)₃ | 70 |
| *4h* | OMe | OMe | (CH₂)₄ | 86 |
| *4i* | OMe | OMe | (CH₂)₅ | - |
| *4j* | H | H | *o*-xylene | 19 |
| *4k* | H | H | *m*-xylene | 29 |
| *4l* | H | H | *p*-xylene | 27 |
| *4m* | OMe | H | *o*-xylene | 62 |
| *4n* | OMe | H | *m*-xylene | 74 |
| *4o* | OMe | H | *p*-xylene | 90 |
| *4p* | OMe | OMe | *o-*xylene | 28 |
| *4q* | OMe | OMe | *m*-xylene | 60 |
| *4r* | OMe | OMe | *p*-xylene | 51 |

| | | | | |
|---|---|---|---|---|
| ^{a}% of ¹²⁵I-apamin displaced at 10 µM | | | | |

In table 1, the screening experiment shows that different bis 1,2,3,4-tetrahydroisoquinoline derivatives possess a higher potential compared to *N-*Methyl-Laudanosine (NML) to interact with the apamin-sensitive sites. The % of ¹²⁵I-apamin displaced at 10 µM is equivalent and sometime higher for some bis-(1,2,3,4)-tetrahydroisoquinoline derivatives. The compounds presenting a value of ¹²⁵I-apamin displaced at 10 µM superior to 70% are selected for precise determination of the affinity (Ki) (see table 2 and 3).

**Table 2: Binding Affinities of stereoisomers of bis-1,2,3,4-TetrahydroIsoquinoline Alkane Derivatives (4d-h) for Rat Cortical Apamin Sensitive Sites in Comparison with N-Methyl-Laudanosine (NML) and Dequalinium (DQ+).**

| | | | | |
|---|---|---|---|---|
| 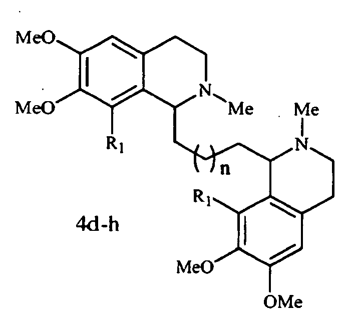 | | | | |

| *N*° | *stereo-isomers^{a}* | *R₁* | *n* | *Kᵢ (nM)* |
|---|---|---|---|---|
| *NML* | | - | - | 1295±15 |
| *DQ+* | | - | - | 221±11 |
| *4d* | E1 | H | 1 | 293±22 |
| | MESO | H | 1 | 1422±116 |
| | E2 | H | 1 | 1885±105 |
| *4e* | E1 | H | 2 | 588±72 |
| | MESO | H | 2 | 1069±124 |
| | E2 | H | 2 | 1196±114 |
| *4f* | E1 | H | 3 | 592±14 |
| | MESO | H | 3 | 731±63 |
| | E2 | H | 3 | 1304±39 |
| *4g* | E1 | OMe | 1 | 1739±195 |
| | MESO | OMe | 1 | 1668±77 |
| | E2 | OMe | 1 | 1104±103 |
| *4h* | E1 | OMe | 2 | 2210±156 |
| | MESO | OMe | 2 | 424±33 |
| | E2 | OMe | 2 | 746±136 |

| | | | | |
|---|---|---|---|---|
| ^{a} E1 = first eluted enantiomer and E2 = second eluted enantiomer | | | | |

In table 2 one can observe that several compounds i.e. 4dE1, 4eE1, 4fE1, 4fMESO, 4hMESO and 4hE2 possess a Ki superior to NML or for 4dE1 a Ki quite similar to dequalinium. The great advantage of these original compounds is represented by their basic character, unlike NML or DQ+ which are permanently charged, permitting readily to cross the blood-brain barrier for interacting with central sites. Another interesting point concerns the differential affinity of stereoisomers which suggest a preferential configuration for interacting with the appropriate binding sites.

**Table 3: Binding Affinities of stereoisomers of bis-1,2,3,4-TetrahydroIsoquinoline Xylene Derivatives (4m-n) for Rat Cortical Apamin Sensitive Sites in Comparison with N-Methyl-Laudanosine (NML) and Dequalinium (DQ+).**

| | | | |
|---|---|---|---|
| 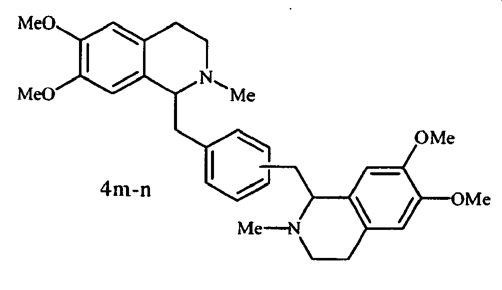 | | | |

| *N*° | *stereo-isomers^{a}* | *xylene substitution* | *Kᵢ (nM)* |
|---|---|---|---|
| *NML* | - | - | 1295±15 |
| *DQ+* | - | - | 221±11 |
| *4m* | E1 | *meta* | 1279±55 |
| | MESO | *meta* | 1524±99 |
| | E2 | *meta* | 1020±27 |
| *4n* | E1 | *para* | 1013±144 |
| | MESO | *para* | 566±26 |
| | E2 | *para* | 1123±46 |

| | | | |
|---|---|---|---|
| ^{a} E1 = first eluted enantiomer and E2 = second eluted enantiomer | | | |

In table 3, compound 4mMESO presents a higher potential than NML to interact with the apamin-sensitive sites of the SK channels.

## Claims

1. Bis 1,2,3,4-tetrahydroisoquinoline derivatives selected from
1,3-Bis[1-(2-methyl-1,2,3,4-tetrahydrolisoquinolyl)]-propane hydrochloride;
1,4-Bis[1-(2-mrmyl-1,2,3,4-tetrahydroisoquinolyl)]-butane hydrochloride;
1,5-Bis[1-(2-methyl-1,2,3,4-tetrahydroisoquinolyl)]-pentane hydrochloride;
1,3-Bis[1-(6,7-dimethoxy-2-methyl-1,2,3,4-tetrahydroisoquinolyl)]-propane hydrochloride ;
1,4-Bis[1-(6,7-dimethoxy-2-methyl-1,2,3,4-tetrahydroisoquinolyl)]-butane hydrochloride ;
1,5-Bis[1-(6,7-dimethoxy-2-methyl-1,2,3,4-tetrahydroisoquinolyl)]-pentane hydrochloride ;
1,3-Bis[1-(6,7,8-trimethoxy-2-methyl-1,2,3 ,4-tetrahydroisoquinolyl)]-propane hydrochloride ;
1,4-Bis[1-(6,7,8-trimethoxy-2-methyl-1,2,3,4-tetrahydxoisoduinolyl)]-butane hydrochloride ;
o-Bis[1-(2-methyl-1,2,3,4-tetrahydroisoquinolyl)]-xylene hydrochloride ;
m-Bis[1-(2-methyl-1,2,3,4-tetrahydroisoquinolyl)]-xylene hydrochloride ;
p-Bis[1-(2-methyl-1,2,3,4-tetrahydroisoquinolyl)]-xylene hydrochloride ;
o-Bis[1-(6,7-dimethoxy-2-methyl-1,2,3,4-tetrahydroxsoquinolyl)]-xylene hydrochloride ;
m-Bis [1-(6,7-dimethoxy-2-methyl-1,2,3,4-tetrahydroisoquinolyl)]-xylene hydrochloride ;
p-Bis[1-(b,7-dimethoxy-2-methyl-1,2,3,4-tetrahydroisoquinolyl)]-xylene ;
o-Bis[1-(6,7,8-trim,ethoxy-2-methyl-1,2,3,4-tetrahydroisoquinolyl)]-xylene hydrochloride ;
m-Bis[1-(6,7,8-trimethoxy-2-methyl-1,2,3,4-tetrahydroxsoquinolyl)]-xylene hydrochloride
p-Bis[1-(6,7,8-trimethoxy-2-methyl-1,2,3,4-tetrahydroisoquinolyl)]-xylene;
1,3-bis[1-(2,8-dimethyl-1,2,3,4-tetrahydroisoquinolyl)]-propane hydrochloride;
1,3-bis[1-(5-bromo-2-methyl-1,2,3,4- tetrahydroisoquinolyl)]-propane hydrochloride; and
1,1'-bis[1-(2-methyl-1,2,3,4-tetrahydroisoquinolyl)]-dimethylether.

2. Bis-1,2,3,4-tetrahydroisoquinoline derivatives according to claim 1 for use as medicament.

3. A pharmaceutical composition comprising an effective amount of bis 1,2,3,4-tetrahydroisoquinoline derivatives according to claim 1 or a pharmaceutical acceptable salt thereof as well as a pharmaceutically acceptable diluent or carrier.

4. Use of bis-1,2,3,4-tetrahydroisoquinoline derivatives of formula (I), any stereoisomer thereof, including optical isomer or mixture of optical isomers thereof, including racemic mixture or any tautomeric or polymorphic form thereof and their pharmaceutically acceptable salt thereof, 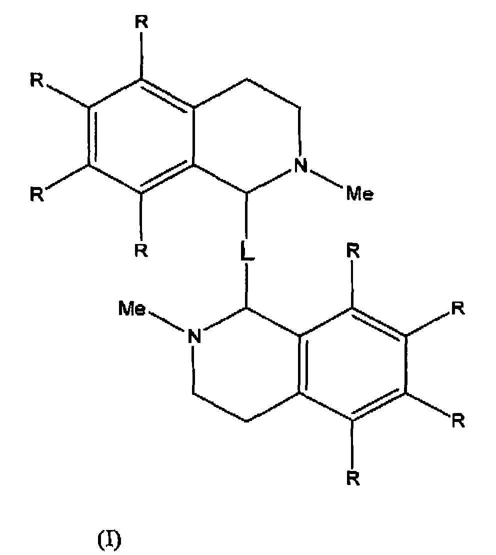 wherein
R which may be the same or different, represents hydrogen, hydroxy, C₁₋₁₂ alkyl, C₁₋₁₂ alkoxy, C₁₋₁₂ alkylthio, a C₁₋₁₂ alkylsulfoxide, a C₁₋₁₂ alkyl-sulfone, C₂₋₆ alkylenedioxy, an amino, an amido, an azido, nitro, C₁₋₁₂ alkylamino, C₁₋₁₂ alkylamido, C₁₋₁₂ alkylsulfonamido, a perhaloalkyl, a carboxyalkyl, a carboxy, a carbamide, dialkylamino, an aryl or halogen;
and L represents a C₁₋₁₂-alkyl, C₂₋₁₂₋alkenyl, a C₂₋₁₂₋alkynyl, an aryl, a diaryl, a cycloalkyl, an heterocycloalkyl, a cycloalkene, an heterocycloalkenyl, an heteroaryl, an ether, a thioether, a sulfoxide, a sulfone, a urea, a thiourea or a guanidine, for the manufacture of a medicament for the treatment of diseases of the Central Nervous System.

5. Use of bis 1,2,3,4-tetrahydroisoquinoline derivatives of formula (I) according to claim 4, wherein
R represents hydrogen, hydroxy, C₁₋₆ alkyl, C₁₋₆ alkoxy or an halogen;
and L represents C₁₋₆alkyl, C₂₋₆alkenyl, a C₂₋₆alkynyl, an aryl, a bis (metbylene)-cycloalkyl or a bis(methylene)cycloalkene.

6. Use of bis 1,2,3,4-tetrahydroisoquinoline derivatives of formula (I) according to claim 4, wherein
R represents hydrogen, hydroxy, C₁₋₆₋alkyl or C₁₋₆alkylthio; and
L represents C₁₋₆₋alkyl, C₂₋₆₋alkenyl, a C₂₋₆₋alkynyl, an aryl, a bis (methylene)-cycloalkyl or a bis(methylene)cycloalkene.

7. Use of bis 1,2,3,4-tetrahydroisoquinoline derivatives of formula (I) according to claim 4 wherein said derivatives are selected from
1,3-Bis[1-(2-methyl-1,2,3,4-tetrahydrolisoquinolyl)]-propane hydrochloride;
1,4-Bis[1-(2-methyl-1,2,3,4-tetrahydroisoquinolyl)]-butane hydrochloride;
1,5-Bis[1-(2-methyl-1,2,3,4-tetrahydroisoquinolyl)]-pentane hydrochloride ;
1,3-Bis[1-(6,7-dimethoxy-2-methyl-1,2,3,4-tetrahydroisoquinolyl)]-propane hydrochloride ;
1,4-Bis[1-(6,7-dimethoxy-2-methyl-1,2,3,4-tetrahydroisoquinolyl)]-butane hydrochloride ;
1,5-Bis[1-(6,7-dimethoxy-2-methyl-1,2,3,4-tetrahydroisoquinolyl)]-pentane hydrochloride;
1,3-Bis[1-(6,7,8-trimethoxy-2-methyl-1,2,3,4-tetrahydroisoquinolyl)]-propane hydrochloride ;
1,4-Bis[1-(6,7,8-trimethoxy-2-methyl-1,2,3,4-tetrahydroisoquinolyl)]-butane hydrochloride ;
o-Bis[1-(2-methyl-1,2,3,4-tetrahydroisoquinolyl)]-xylene hydrochloride;
m-Bis[1-(2-methyl-1,2,3,4-tetrahydroisoquinolyl)]-xylene hydrochloride ;
p-Bis[1-(2-methyl-1,2,3,4-tetrahydroisoquinolyl)]-xylene hydrochloride;
o-Bis[1-(6,7-dimethoxy-2-methyl-1,2,3,4-tetrahydroisoquinolyl)]-xylene hydrochloride ;
m-Bis[1-(6,7-dimethoxy-2-metbyl-1,2,3,4-tetrahyldroisoquinolyl)]-xylene hydrochloride ;
p-Bis[1-(6,7-dimethoxy-2-methyl-1,2,3,4-tetrahydroisoquinolyl)]-xylene;
o-Bis[1-(6,7,8-trimethoxy-2-methyl-1,2,3,4-tetrahydroisoquinolyl)]-xylene hydrochloride ;
m-Bis[1-(6,7,8-trimethoxy-2-methyl-1,2,3,4-tetrahydroisoquinolyl)]-xylene hydrochloride
p-Bis[1-(6,7,8-trimethoxy-2-methyl-1,2,3,4-tetrahydroisoquinolyl)]-xylene;
1,3-bis[1-(2,8-dimethyl-1,2,3,4-tetrahydroisoquinolyl)]-propane hydrochloride;
1,3-bis[1-(5-bromo-2-methyl-1,2,3,4-tetrahydroisoquinolyl)]-propane hydrochloride; and
1,1'-bis[1-(2-methyl-1,2,3,4-tetrahydroisoquinolyl)]-dimethylether.

8. Use of bis 1,2,3,4-tetrahydroisoquinoline derivative of formula (I) according to any one of claims 4 to 7 wherein the disease of the Central Nervous System is a neurogenerative disorder, preferably Parkinson's disease.

9. Use of bis 1,2,3,4-tetrahydroisoquinoline derivative of formula (I) according to any one of claims 4 to 7 wherein the disease of the Central Nervous System is a psychotic disorder preferably Alzheimer's disease or schizophrenia.

## Patentansprüche

1. Bis-1,2,3,4-tetrahydroisochinolin-Derivate, ausgewählt unter
1,3-Bis[1-(2-methyl-1,2,3,9-tetrahydroisochinolyl)]propan-hydrochlorid;
1,4-Bis[1-(2-methyl-1,2,3,4-tetrahydroisochinolyl)]butan-hydrochlorid;
1,5-Bis[1-(2-methyl-1,2,3,9-tetrahydroisochinolyl)]pentan-hydrochlorid;
1,3-Bis[1-(6,7-dimethoxy-2-methyl-1,2,3,4-tetrahydroisochinolyl)]propan-hydrochlorid;
1,4-Bis[1-(6,7-dimethoxy-2-methyl-1,2,3,4-tetrahydroisochinolyl)]butan-hydrochlorid;
1,5-Bis[1-(6,7-dimethoxy-2-methyl-1,2,3,4-tetrahydroisochinolyl)]pentan-hydrochlorid;
1,3-Bis[1-(6,7,8-trimethoxy-2-methyl-1,2,3,4-tetrahydroisochinolyl)]propan-hydrochlorid;
1,4-Bis[1-(6,7,8-trimethoxy-2-methyl-1,2,3,9-tetrahydroisochinolyl)]butan-hydrochlorid;
o-Bis[1-(2-methyl-1,2,3,9-tetrahydroisochinolyl)]-xylol-hydrochlorid;
m-Bis[1-(2-methyl-1,2,3,4-tetrahydroisochinolyl)]-xylol-hydrochlorid;
p-Bis[1-(2-methyl-1,2,3,4-tetrahydroisochinolyl)]-xylol-hydrochlorid;
o-Bis[1-(6,7-dimethoxy-2-methyl-1,2,3,4-tetrahydroisochinolyl)]xylol-hydrochlorid;
m-Bis[1-(6,7-dimethoxy-2-methyl-1,2,3,4-tetrahydroisochinolyl)]xylol-hydrochlorid;
p-Bis[1-(6,7-dimethoxy-2-methyl-1,2,3,4-tetrahydroisochinolyl)]xylol;
o-Bis[l-(6,7,8-trimethoxy-2-methyl-1,2,3,4-tetrahydroisochinolyl)]xylol-hydrochlorid;
m-Bis[1-(6,7,8-trimethoxy-2-methyl-1,2,3,4-tetrahydroisochinolyl)]xylol-hydrochlorid;
p-Bis[1-(6,7,8-tximethoxy-2-methyl-1,2,3,4-tetrahydroisochinolyl)]xylol;
1,3-Bis[1-(2,8-dimethyl-1,2,3,4-tetrahydroisochinolyl)]propan-hydrochlorid;
1,3-Bis[1-(5-brom-2-methyl-1,2,3,4-tetrahydroisochinolyl)]propan-hydrochlorid und
1,1'-Bis[1-(2-methyl-1,2,3,4-tetrahydroisochinolyl)]dimethylether.

2. Bis-1,2,3,4-tetrahydroisochinolin-Derivate nach Anspruch 1 zur Verwendung als Arzneimittel.

3. Pharmazeutische Zusammensetzung, enthaltend eine wirksame Menge von Bis-1,2,3,4-tetrahydroisochinolin-Derivaten nach Anspruch 1 oder ein pharmazeutisch unbedenkliches Salz davon sowie ein pharmazeutisch unbedenkliches Verdünnungsmittel oder einen pharmazeutisch unbedenklichen Träger.

4. Verwendung von Bis-1,2,3,4-tetrahydroisochinolin-Derivaten der Formel (I), eines Stereoisomers davon, einschließlich eines optischen Isomers oder eines Gemischs von optischen Isomeren davon, einschließlich racemischer Gemische oder einer tautomeren oder polymorphen Form davon und ihrer pharmazeutisch unbedenklichen Salze davon, 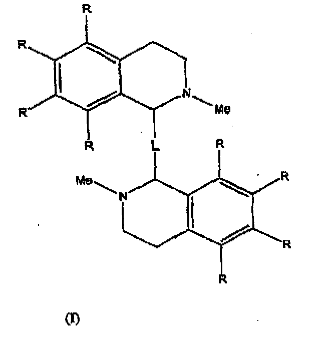 worin
R gleich oder verschieden sein kann und für Wasserstoff, Hydroxy, C₁₋₁₂-Alkyl, C₁₋₁₂-Alkoxy, C₁₋₁₂-Alkylthio, ein C₁₋₁₂-Alkylsulfoxid, ein C₁₋₁₂-Alkylsulfon, C₂₋₆-Alkylendioxy, ein Amino, ein Amido, ein Azido, Nitro, C₁₋₁₂-Alkylamino, C₁₋₁₂-Alkylamido, C₁₋₁₂-Alkylsulfonamido, ein Perhalogenalkyl, ein Carboxyalkyl, ein Carboxy, ein Carbamid, Dialkylamino, ein Aryl oder Halogen steht
und L für ein C₁₋₁₂-Alkyl, C₂₋₁₂-Alkenyl, ein C₂₋₁₂-Alkinyl, ein Aryl, ein Diaryl, ein Cycloalkyl, ein Heterocycloalkyl, ein Cycloalken, ein Heterocycloalkenyl, ein Heteroaryl, einen Ether, einen Thioether, ein Sulfoxid, ein Sulfon, einen Harnstoff, einen Thioharnstoff oder ein Guanidin steht, zur Herstellung eines Arnzeimittels für die Behandlung von Erkrankungen des Zentralnervensystems.

5. Verwendung von Bis-1,2,3,4-tetrahydroisochinolin-Derivaten der Formel (I) nach Anspruch 4, worin
R für Wasserstoff, Hydroxy, C₁₋₆-Alkyl, C₁₋₆-Alkoxy oder ein Halogen steht und
L für C₁₋₆-Alkyl, C₂₋₆-Alkenyl, ein C₂₋₆-Alkinyl, ein Aryl, ein Bis(methylen)cycloalkyl oder ein Bis-(methylen)cycloalken steht.

6. Verwendung von Bis-1,2,3,4-tetrahydroisochinolin-Derivaten der Formel (I) nach Anspruch 4, worin
R für Wasserstoff, Hydroxy, C₁₋₆-Alkyl oder C₁₋₆-Alkylthio steht und
L für C₁₋₆-Alkyl, C₂₋₆-Alkenyl, ein C₂₋₆-Alkinyl, ein Acryl, ein Bis(methylen)cycloalkyl oder ein Bis-(methylen)cycloalken steht.

7. Verwendung von Bis-1,2,3,4-tetrahydroisochinolin-Derivaten der Formel (I) nach Anspruch 4, wobei die Derivate unter
1,3-Bis[1-(2-methyl-1,2,3,4-tetrahydroisochinolyl)]propan-hydrochlorid;
1,4-Bis[1-(2-methyl-1,2,3,4-tetrahydroisochinolyl)]butan-hydrochlorid;
1,5-Bis[1-(2-methyl-1,2,3,4-tetrahydroisochinolyl)]pentan-hydrochlorid;
1,3-Bis[1-(6,7-dimethoxy-2-methyl-1,2,3,4-tetrahydroisochinolyl)]propan-hydrochlorid;
1,4-Bis[1-(6,7-dimethoxy-2-methyl-1,2,3,4-tetrahydroisochinolyl)]butan-hydrochlorid;
1,5-Bis[1-(6,7-dimethoxy-2-methyl-1,2,3,4-tetrahydroisochinolyl)]pentan-hydrochlorid;
1,3-Bis[1-(6,7,8-trimethoxy-2-methyl-1,2,3,4-tetrahydroisochinolyl)]propan-hydrochlorid;
1,4-Bis[1-(6,7,8-trimethoxy-2-methyl-1,2,3,4-tetrahydroisochinolyl)]butan-hydrochlorid;
o-Bis[1-(2-methyl-1,2,3,4-tetrahydroisochinolyl)]-xylol-hydrochlorid;
m-Bis[1-(2-methyl-1,2,3,4-tetrahydroisochinolyl)]-xylol-hydrochlorid;
p-Bis[1-(2-methyl-1,2,3,4-tetrahydroisochinolyl)]-xylol-hydrochlorid;
o-Bis[1-(6,7-dimethoxy-2-methyl-1,2,3,4-tetrahydroisochinolyl)]xylol-hydrochlorid;
m-Bis[1-(6,7-dimethoxy-2-methyl-1,2,3,4-tetrahydroisochinolyl)]xylol-hydrochloxid;
p-Bis[1-(6,7-dimethoxy-2-methyl-1,2,3,4-tetrahydroisochinolyl)]xylol:
o-Bis[1-(6,7,8-trimethoxy-2-methyl-1,2,3,4-tetrahydroisochinolyl)]xylol-hydrochlorid;
m-Bis[1-(6,7,8-trimethoxy-2-methyl-1,2,3,4-tetrahydroisochinolyl)]xylol-hydrochlorid;
p-Bis[1-(6,7,8-trimethoxy-2-methyl-1,2,3,4-tetrahydroisochinolyl)]xylol;
1,3-Bis[1-(2,8-dimethyl-1,2,3,4-tetrahydroisochinolyl)]propan-hydrochlorid;
1,3-Bis[1-(5-brom-2-methyl-1,2,3,4-tetrahydroisochinolyl)]propan-hydrochlorid und
1,1'-Bis[1-(2-methyl-1,2,3,4-tetrahydroisochinolyl)]dimethylether
ausgewählt sind.

8. Verwendung von Bis-1,2,3,4-tetrahydroisochinolin-Derivaten der Formel (I) nach einem der Ansprüche 4 bis 7, wobei es sich bei der Erkrankung des Zentralnervensystems um eine neurodegenerative Störung, vorzugsweise Parkinson-Krankheit, handelt.

9. Verwendung von Bis-1,2,3,4-tetrahydroisochinolin-Derivaten der Formel (I) nach einem der Ansprüche 4 bis 7, wobei es sich bei der Erkrankung des Zentralnervensystems um eine psychotische Störung, vorzugsweise Alzheimer-Krankheit oder Schizophrenie, handelt.

## Revendications

1. Dérivés de bis 1,2,3,4-tétrahydroisoquinoline choisis parmi les produits suivants :
Chlorhydrate de 1,3-bis[1-(2-méthyl-1,2,3,4-tétrahydroisoquinolyl)]-propane ;
Chlorhydrate de 1,4-bis[1-(2-méthyl-1,2,3,4-tétrahydroisoquinolyl)]-butane ;
Chlorhydrate de 1,5-bis[1-(2-méthyl-1,2,3,4-tétrahydroisoquinolyl)]-pentane ;
Chlorhydrate de 1,3-bis[1-(6,7-diméthoxy-2-méthyl-1,2,3,4-tétrahydroisoquinolyl)]-propane ;
Chlorhydrate de 1,4-bis[1-(6,7-diméthoxy-2-méthyl-1,2,3,4-tétrahydroisoquinolyl)]-butane ;
Chlorhydrate de 1,5-bis[1-(6,7-diméthoxy-2-méthyl-1,2,3,4-tétrahydroisoquinolyl)]-pentane ;
Chlorhydrate de 1,3-bis[1-(6,7,8-triméthoxy-2-méthyl-1,2,3,4-tétrahydroisoquinolyl)]-propane ;
Chlorhydrate de 1,4-bis[1-(6,7,8-triméthoxy-2-méthyl-1,2,3,4-tétrahydroisoquinolyl)]-butane ;
Chlorhydrate de o-bis[1-(2-méthyl-1,2,3,4-tétrahydroisoquinolyl)]-xylène ;
Chlorhydrate de m-bis[1-(2-méthyl-1,2,3,4-tétrahydroisoquinolyl)]-xylène ;
Chlorhydrate de p-bis[1-(2-méthyl-1,2,3,4-tétrahydroisoquinolyl)]-xylène ;
Chlorhydrate de o-bis[1-(6,7-diméthoxy-2-méthyl-1,2,3,4-tétrahydroisoquinolyl)]-xylène ;
Chlorhydrate de m-bis[1-(6,7-diméthoxy-2-méthyl-1,2,3,4-tétrahydroisoquinolyl)]-xylène ;
Chlorhydrate de p-bis[1-(6,7-diméthoxy-2-méthyl-1,2,3,4-tétrahydroisoquinolyl)]-xylène ;
Chlorhydrate de o-bis[1-(6,7,8-triméthoxy-2-méthyl-1,2,3,4-tétrahydroisoquinolyl)]-xylène ;
Chlorhydrate de m-bis[1-(6,7,8-triméthoxy-2-méthyl-1,2,3,4-tétrahydroisoquinolyl)]-xyléne :
Chlorhydrate de p-bis[1-(6,7,8-triméthoxy-2-méthyl-1,2,3,4-tétrahydroisoquinolyl)]-xylène ;
Chlorhydrate de 1,3-bis[1-(2,8-diméthyl-1,2,3,4-tétrahydroisoquinolyl)]-propane ;
Chlorhydrate de 1,3-bis[1-(5-bromo-2-méthyl-1,2,3,4-tétrahydroisoquinolyl)]-propane ;
et
1,1'-bis[1-(2-méthyl-1,2,3,4-tétrahydroisoquinolyl)]-diméthyléther.

2. Dérivés de bis 1,2,3,4-tétrahydroisoquinoline selon la revendication 1 pour utilisation comme médicament.

3. Composition pharmaceutique comprenant une quantité efficace de dérivés de bis 1,2,3,4-tétrahydroisoquinoline selon la revendication 1 ou d'un sel pharmaceutiquement acceptable de ceux-ci, ainsi qu'un diluant ou un vecteur pharmaceutiquement acceptable.

4. Utilisation de dérivés bis-1,2,3,4-tétrahydroisoquinoline de la formule (I), de tout stéréo-isomère de ceux-ci, y compris un isomère optique ou un mélange d'isomères optiques de ceux-ci, y compris un mélange racémique ou toute forme tautomère ou polymorphe de ceux-ci et les sels pharmaceutiquement acceptables de ceux-ci, 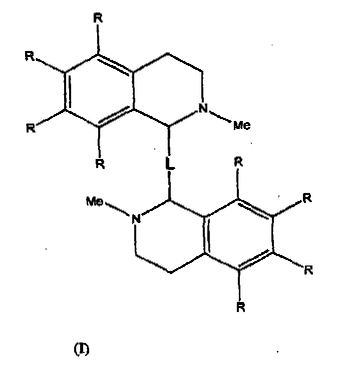 dans laquelle
R, qui peut être identique ou différent, représente un atome d'hydrogène, un groupe hydroxy, alkyle de C₁ à C₁₂, alcoxy de C₁ à C₁₂, alkyle de C₁ à C₁₂-thio, alkyle de C₁ à C₁₂-sulfoxyde, alkyle de C₁ à C₁₂-sulfone, alkylène de C₂ à C₆-dioxy, amino, amido, azido, nitro, alkyle de C₁ à C₁₂-amino, alkyle de C₁ à C₁₂-amido, alkyle de C₁ à C₁₂-sulfonamido, perhaloalkyle, carboxyalkyle, carboxy, carbamide, dialkylamino, aryle ou halogène ;
et L représente un groupe alkyle de C₁ à C₁₂, alcényle de C₂ à C₁₂, alcynyle de C₂ à C₁₂, aryle, diaryle, cycloalkyle, hétérocycloalkyle, cycloalcène, hétérocycloalcényle, hétéroaryle, éther, thioéther, sulfoxyde, sulfone, urée, thio-urée ou guanidine, pour la fabrication d'un médicament pour le traitement de maladies du système nerveux central.

5. Utilisation de dérivés de bis 1,2,3,4-tétrahydroisoquinoline de la formule (I) selon la revendication 4, dans laquelle
R représente un atome d'hydrogène, un groupe hydroxy, alkyle de C₁ à C₆, alcoxy de C₁ à C₆ ou un atome d'halogène ; et
L représente un groupe alkyle de C₁ à C₈, alcényle de C₂ à C₆, alcynyle de C₂ à C₆, aryle, bis(méthylène)-cycloalkyle ou bis(méthylène)cycloalcène.

6. Utilisation de dérivés de bis 1,2,3,4-tétrahydroisoquinoline de la formule (I) selon la revendication 4, dans laquelle
R représente un atome d'hydrogène, un groupe hydroxy, alkyle de C₁ à C₆ ou alkyle de C₁ à C₆-thio ; et
L représente un groupe alkyle de C₁ à C₆, alcényle de C₂ à C₆, alcynyle de C₂ à C₆, aryle, bis(méthylène)-cycloalkyle ou bis(méthylène)cycloalcène.

7. Utilisation de dérivés de bis 1,2,3,4-tétrahydroisoquinoline de la formule (I) selon la revendication 4, dans laquelle lesdits dérivés sont choisis parmi les produits suivants :
Chlorhydrate de 1,3-bis[1-(2-méthyl-1,2,3,4-tétrahydroisoquinolyl)]-propane ;
Chlorhydrate de 1,4-bis[1-(2-méthyl-1,2,3,4-tétrahydroisoquinolyl)]-butane ;
Chlorhydrate de 1,5-bis[1-(2-méthyl-1,2,3,4-tétrahydroisoquinolyl)]-pentane ;
Chlorhydrate de 1,3-bis[1-(6,7-diméthoxy-2-méthyl-1,2,3,9-tétrahydroisoquinolyl)]-propane :
Chlorhydrate de 1,4-bis[1-(6,7-diméthoxy-2-méthyl-1,2,3,4-tétrahydroisoquinolyl)]-butane :
Chlorhydrate de 1,5-bis[1-(6,7-diméthoxy-2-méthyl-1,2,3,4-tétrahydroisoquinolyl)]-pentane ;
Chlorhydrate de 1,3-bis[1-(6,7,8-triméthoxy-2-méthyl-1,2,3,4-tétrahydroisoquinolyl)]-propane ;
Chlorhydrate de 1,4-bis[1-(6,7,8-triméthoxy-2-méthyl-1,2,3,4-tétrahydroisoquinolyl)]-butane ;
Chlorhydrate de o-bis[1-(2-méthyl-1,2,3,4-tétrahydroisoquinolyl)]-xylène ;
Chlorhydrate de m-bis[1-(2-méthyl-1,2,3,4-tétrahydroisoquinolyl)]-xylène ;
Chlorhydrate de p-bis[1-(2-méthyl-1,2,3,4-tétrahydroisoquinolyl)]-xylène ;
Chlorhydrate de o-bis[1-(6,7-diméthoxy-2-méthyl-1,2,3,4-tétrahydroisoquinolyl)]-xylène ;
Chlorhydrate de m-bis[1-(6,7-diméthoxy-2-méthyl-1,2,3,4-tétrahydroisoquinolyl)]-xylène ;
Chlorhydrate de p-bis[1-(6,7-diméthoxy-2-méthyl-1,2,3,4-tétrahydroisoquinolyl)]-xylène ;
Chlorhydrate de o-bis[1-(6,7,8-triméthoxy-2-méthyl-1,2,3,4-tétrahydroisoquinolyl)]-xyléne ;
Chlorhydrate de m-bis[1-(6,7,8-triméthoxy-2-méthyl-1,2,3,4-tétrahydroisoquinolyl)]-xylène ;
Chlorhydrate de p-bis[1-(6,7,8-trimêthoxy-2-méthyl-1,2,3,4-tétrahydroisoquinolyl)]-xylène ;
Chlorhydrate de 1,3-bis[1-(2,8-diméthyl-1,2,3,4-tétrahydroisoquinolyl)]-propane :
Chlorhydrate de 1,3-bis[1-(5-bromo-2-méthyl-1,2,3,4-tétrahydroisoquinolyl)]-propane ;
et
1,1'-bis[1-(2-méthyl-1,2,3,4-tétrahydroisoquinolyl)]-diméthyléther.

8. Utilisation de dérivés de bis 1,2,3,4-tétrahydroisoquinoline de la formule (I) selon l'une quelconque des revendications 4 à 7, dans laquelle la maladie du système nerveux central est une affection neurodégénérative, de préférence la maladie de Parkinson.

9. Utilisation de dérivés de bis 1,2,3,4-tétrahydroisoquinoline de la formule (I) selon l'une quelconque des revendications 4 à 7, dans laquelle la maladie du système nerveux central est un trouble psychotique, de préférence la maladie d'Alzheimer ou la schizophrénie.
